# FASCICULE DE BREVET EUROPEEN

(11) **EP 0 673 372 B1**
(45) Date de publication et mention de la délivrance du brevet: **25.06.2003**
(21) Numéro de dépôt: 94901993.9
(22) Date de dépôt: 07.12.1993
(51) Int. Cl.: C07D 305/14, A61K 31/335

(54) **NOUVEAUX TAXOIDES, LEUR PREPARATION ET LES COMPOSITIONS PHARMACEUTIQUES QUI LES CONTIENNENT**
TAXOL DERIVATE, DEREN HERSTELLUNG UND DIESE ENTHALTENDE PHARMAZEUTISCHE ZUSAMMENSETZUNGEN
NOVEL TAXOIDS, PREPARATION THEREOF AND PHARMACEUTICAL COMPOSITIONS CONTAINING SAME

(30) Priorité: 09.12.1992 FR 9214813
(43) Date de publication de la demande: 27.09.1995
(73) Titulaire: Aventis Pharma S.A., 92160 Antony (FR)
(72) Inventeur: BOUCHARD, Hervé, F-94200 Ivry-sur-Seine (FR); BOURZAT, Jean-Dominique, F-94300 Vincennes (FR); COMMERCON, Alain, F-94400 Vitry-sur-Seine (FR)
(74) Mandataire: Savina, Jacques
(86) Numéro de dépôt international: FR9301201
(87) Numéro de publication internationale: WO94013654

(56) Documents cités:
- WO-A-95/13271
- JOURNAL OF MEDICINAL CHEMISTRY vol. 34 , 1991 , WASHINGTON US pages 992 - 998 F. GUERITTE-VOEGELEIN ET AL. 'RELATIONSHIP BETWEEN THE STRUCTURE OF TAXOL ANALOGUES AND THEIR ANTIMITOTIC ACTIVITY.'

## Description

La présente invention concerne de nouveaux taxoïdes de formule générale : 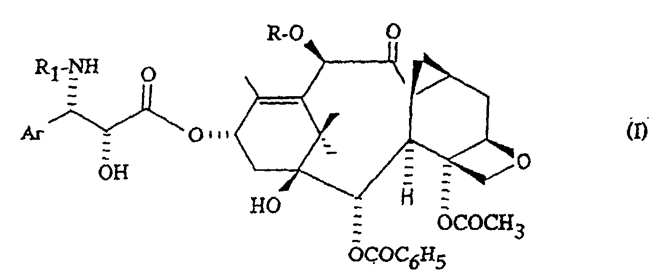 leur préparation et les compositions pharmaceutiques qui les contiennent.

Dans la formule générale (I),
Ar représente un radical aryle,
R représente un atome d'hydrogène ou un radical acétyle, alcoxyacétyle ou alcoyle,
R₁ représente un radical benzoyle ou un radical R₂-O-CO- dans lequel R₂ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie la partie alcoyle contient 1 à 4 atomes de carbone,
- ou un radical phényle éventuellement substitué par un ou plusieurs atomes ou radicaux choisis parmi les atomes d'halogène et les radicaux alcoyles contenant 1 à 4 atomes de carbone, alcoyloxy contenant 1 à 4 atomes de carbone,
- ou un radical hétérocyclyle azoté saturé ou non saturé contenant 4 à 6 chaînons et éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone.

De préférence Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoyithio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles, ou bien Ar représente un radical hétérocyclique aromatique ayant 5 chaînons et contenant un ou plusieurs atomes, identiques ou différents, choisis parmi les atomes d'azote, d'oxygène ou de soufre, éventuellement substitué par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

Plus particulièrement, Ar représente un radical phényle, thiényle-2 ou -3 ou furyle-2 ou -3 éventuellement substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino et trifluorométhyle.

Plus particulièrement encore, Ar représente un radical phényle éventuellement substitué par un atome de chlore ou de fluor, ou par un radical alcoyle (méthyle), alcoxy (méthoxy), dialcoylamino (diméthylamino), acylamino (acétylamino) ou alcoxycarbonylamino (tert-butoxycarbonylamino) ou thiényle-2 ou -3 ou furyle-2 ou -3.

D'un intérêt encore plus particulier sont les produits de formule générale (I) dans laquelle Ar représente un radical phényle et R₁ représente un radical benzoyle ou tert.butoxycarbonyle.

Selon la présente invention, les nouveaux taxoïdes de formule générale (I) peuvent être obtenus à partir d'un produit de formule générale : 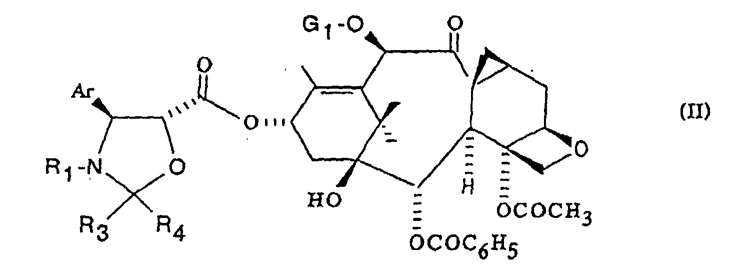 dans laquelle Ar et R₁ sont définis comme précédemment et R₃ et R₄, identiques ou différents représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₃ représente un radical alcoxy contenant 1 à 4 atomes de carbone ou un radical trihalométhyle tel que trichlorométhyle ou un radical phényle substitué par un radical trihalométhyle tel que trichlorométhyle et R₄ représente un atome d'hydrogène, ou bien R₃ et R₄ forment ensemble avec l'atome de carbone auquel ils sont liés un cycle ayant 4 à 7 chaînons, et G₁ représente un atome d'hydrogène ou un radical acétyle, alcoxyacétyle ou alcoyle ou un groupement protecteur de la fonction hydroxy, en opérant, selon les significations de R₃ et R₄, de la manière suivante :
1) lorsque R₃ représente un atome d'hydrogène ou un radical alcoxy contenant 1 à 4 atomes de carbone ou un radical aryle éventuellement substitué et R₄ représente un atome d'hydrogène, le produit de formule générale (II) est traité en milieu acide pour obtenir un produit de formule générale : 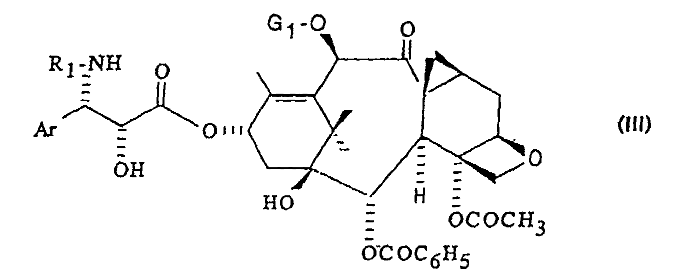 dans laquelle Ar, R₁ et G₁ sont définis comme précédemment, dont le radical G₁ est, si nécessaire, remplacé par un atome d'hydrogène.
   La déprotection de la chaîne latérale du produit de formule générale (II) peut être effectuée en présence d'un acide minéral (acide chlorhydrique, acide sulfurique) ou organique (acide acétique, acide méthanesulfonique, acide trifluorométhanesulfonique, acide p.toluènesulfonique), utilisé seul ou en mélange, en opérant dans un solvant organique choisi parmi les alcools (méthanol, éthanol, isopropanol), les éthers (tétrahydrofuranne, éther diisopropylique, méthyl t-butyléther), les esters (acétate d'éthyle, acétate d'isopropyle, acétate de n.butyle), les hydrocarbures aliphatiques (pentane, hexane, heptane), les hydrocarbures aliphatiques halogènes (dichlorométhane, dichloro-1,2 éthane), les hydrocarbures aromatiques (benzène, toluène, xylènes) et les nitriles (acétonitrile) à une température comprise entre - 10 et 60°C, de préférence entre 15 et 30°C. L'acide plut être utilisé en quantité catalytique, stoechiométrique ou en excès.
   La déprotection peut aussi être réalisée dans des conditions oxydantes en utilisant par exemple le nitrate d'ammonium et de cérium IV dans un mélange acétonitrile-eau ou la dichloro-2,3 dicyano-5,6 benzoquinone-1,4 dans l'eau.
   La déprotection peut être également réalisée dans des conditions réductrices, par exemple par hydrogénolyse en présence d'un catalyseur.
   Lorsque G₁ représente un groupement protecteur, celui-ci est de préférence un radical trichloro-2,2,2 éthoxycarbonyle ou (trichlorométhyl-2 propoxy)-2 carbonyle, dont le remplacement par un atome d'hydrogène est effectué par le zinc, éventuellement associé au cuivre, en présence d'acide acétique à une température comprise entre 20 et 60°C ou au moyen d'un acide minéral ou organique tel que l'acide chlorhydrique ou l'acide acétique en solution dans un alcool aliphatique contenant 1 à 3 atomes de carbone ou dans un ester aliphatique tel que l'acétate d'éthyle, l'acétate d'isopropyle ou l'acétate de n.butyle en présence de zinc éventuellement associé à du cuivre, ou bien, lorsque G₁ représente un radical alcoxycarbonyle, son remplacement éventuel par un atome d'hydrogène s'effectue par traitement en milieu alcalin ou par action d'un halogénure de zinc dans des conditions qui ne touchent pas au reste de la molécule. Généralement, le traitement alcalin est effectué par action de l'ammoniac en milieu hydro-alcoolique à une température voisine de 20°C. Généralement, le traitement par un halogénure de zinc, de préférence l'iodure de zinc est effectué dans le méthanol à une température voisine de 20°C.
2) lorsque R₃ et R₄, identiques ou différents, représentent un radical alcoyle contenant 1 à 4 atomes de carbone, ou un radical aralcoyle dont la partie alcoyle contient 1 à 4 atomes de carbone et la partie aryle est, de préférence un radical phényle, éventuellement substitué, ou bien R₃ représente un radical trihalométhyle ou un radical phényl substitué par un radical trihalométhyle et R₄ représente un atome d'hydrogène, ou bien R₃ et R₄ forment ensemble avec l'atome de carbone auquel ils sont liés un cycle ayant 4 à 7 chaînons, le produit de formule générale (II) est transformé en produit de formule générale : 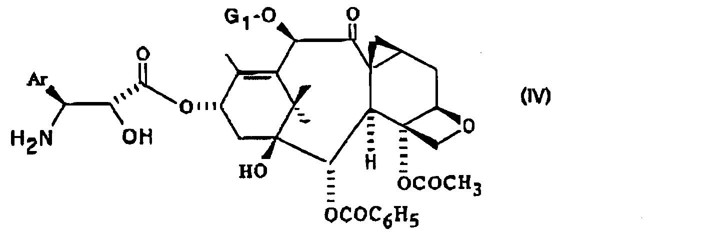 dans laquelle Ar et G₁ sont définis comme précédemment, qui est acylé au moyen de chlorure de benzoyle ou d'un dérivé réactif de formule générale :

   R₂-O-CO-X (V)

   dans laquelle R₂ est défini comme précédemment et X représente un atome d'halogène (fluor, chlore) ou un reste -O-R₂ ou -O-CO-O-R₂, pour obtenir un produit de formule générale (III) dans laquelle Ar, R₁ et G₁ sont définis comme précédemment, dont le radical G₁ est, si nécessaire, remplacé par un atome d'hydrogène.

Les produits de formule générale (IV) peuvent être obtenus en traitant un produit de formule générale (II), dans laquelle Ar, R₁ et G₁ sont définis comme ci-dessus, R₃ et R₄, identiques ou différents, représentent un radical alcoyle, aralcoyle ou aryle, ou bien R₃ et R₄ forment ensemble avec l'atome de carbone auquel ils sont liés un cycle ayant 4 à 7 chaînons, par un acide minéral (acide chlorhydrique, acide sulfurique) ou organique (acide formique) éventuellement dans un alcool contenant 1 à 3 atomes de carbone (méthanol éthanol, isopropanol) à une température comprise entre 0 et 50°C. De préférence, on utilise l'acide formique à une température voisine de 20°C.

L'acylation du produit de formule générale (IV) au moyen de chlorure de benzoyle ou d'un dérivé réactif de formule générale (V) est effectuée dans un solvant organique inerte choisi parmi les esters tels que l'acétate d'éthyle, l'acétate d'isopropyle ou l'acétate de n.bulyle et les hydrocarbures aliphatiques halogénés tels que le dichlorométhane ou le dichloro-1,2 éthane en présence d'une base minérale telle que le bicarbonate de sodium ou organique telle que la triéthylamine. La réaction est effectuée à une température comprise entre 0 et 50°C, de préférence voisine de 20°C,

Lorsque le radical G₁ représente un groupement protecteur, son remplacement par un atome d'hydrogène s'effectue dans les conditions décrites ci-dessus.

Les produits de formule générale (II) peuvent être obtenus selon l'une des méthodes suivantes :

### 1) par estérification du produit de formule générale :

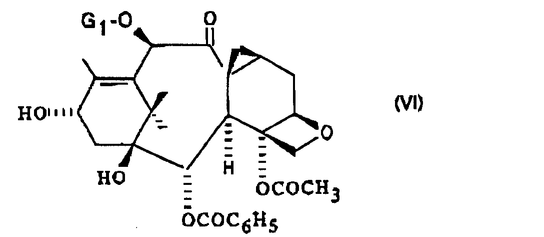

dans laquelle G₁ est défini comme précédemment au moyen d'un acide de formule générale : 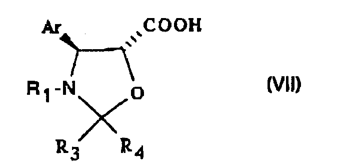 dans laquelle Ar, R₁, R₃ et R₄ sont définis comme précédemment, ou d'un dérivé de cet acide.

L'estérification au moyen d'un acide de formule générale (VII) peut être effectuée en présence d'un agent de condensation (carbodiimide, carbonate réactif) et d'un agent d'activation (aminopyridine) dans un solvant organique (éther, ester, cétones, nitriles, hydrocarbures aliphatiques, hydrocarbures aliphatiques halogénés, hydrocarbures aromatiques) à une température comprise entre -10 et 90°C.

L'estérification peut aussi être réalisée en utilisant l'acide de formule générale (VII) sous forme d'anhydride en opérant en présence d'un agent d'activation (aminopyridine) dans un solvant organique (éthers, esters, cétones, nitriles, hydrocarbures aliphatiques, hydrocarbures aliphatiques halogènes, hydrocarbures aromatiques) à une température comprise entre 0 et 90°C.

L'estérification peut aussi être réalisée en utilisant l'acide de formule générale (VU) sous forme d'halogénure ou sous forme d'anhydride avec un acide aliphatique ou aromatique, éventuellement préparé in situ, en présence d'une base (amine aliphatique tertiaire) en opérant dans un solvant organique (éthers, esters, cétones, nitriles, hydrocarbures aliphatiques, hydrocarbures aliphatiques halogénés, hydrocarbures aromatiques) à une température comprise entre 0 et 80°C.

L'acide de formule générale (VII) peut être obtenu par saponification d'un ester de formule générale : 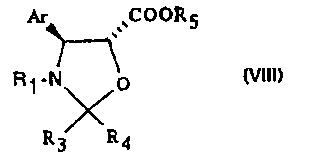 dans laquelle Ar, R₁, R₃ et R₄ sont définis comme précédemment et R₅ représente un radical alcoyle contenant 1 à 4 atomes de carbone éventuellement substitué par un radical phényle.

Généralement, la saponification est effectuée au moyen d'une base minérale (hydroxyde, carbonate ou bicarbonate de métal alcalin) en milieu hydro-alcoolique (méthanol-eau) à une température comprise entre 10 et 40°C.

L'ester de formule générale (VIII) peut être obtenu par action d'un produit de formule générale : 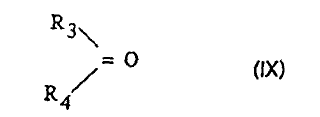 dans laquelle R₃ et R₄ sont définis comme précédemment sous forme d'un dialkylacétal ou d'un alkyléther d'énol, sur un ester de formule générale : 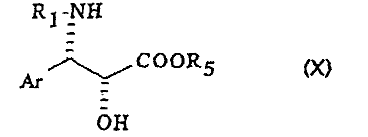 dans laquelle Ar, R₁ et R₅ sont définis comme précédemment en opérant dans un solvant organique inerte (hydrocarbure aromatique) en présence d'un acide fort minéral (acide sulfurique) ou organique (acide p.toluènesulfonique éventuellement sous forme de sel de pyridinium) à une température comprise entre 0°C et la température d'ébullition du mélange réactionnel.

L'ester de formule générale (X) peut être obtenu par action d'un produit de formule générale (V) sur un ester de formule générale : 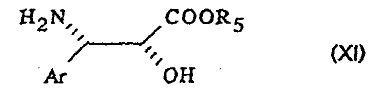 dans laquelle Ar et R₅ sont définis comme précédemment, en opérant dans un solvant organique (ester, hydrocarbure aliphatique halogéné) en présence d'une base minérale ou organique à une température comprise entre 0 et 50°C.

Le produit de formule générale (XI) peut être obtenu par réduction d'un azoture de formule générale : 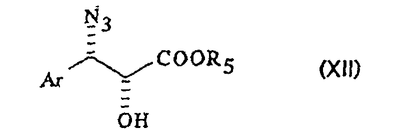 dans laquelle Ar et R₅ sont définis comme précédemment, au moyen d'hydrogène en présence d'un catalyseur tel que le palladium sur noir en opérant dans un solvant organique (ester).

Le produit de formule générale (XII) peut être obtenu par action d'un azoture tel que l'azoture de triméthylsilyle en présence de chlorure de zinc ou azoture de métal alcalin (sodium, potassium, lithium) en milieu hydro-organique (eau-tétrahydrofuranne) à une température comprise entre 20°C et la température d'ébullition du mélange réactionnel sur un époxyde de formule générale : 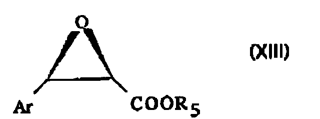 dans laquelle Ar et R₅ sont définis comme précédemment, éventuellement préparé in situ.

L'époxyde de formule générale (XIII) peut être obtenu, éventuellement in situ, par déhydrohalogénation d'un produit de formule générale : 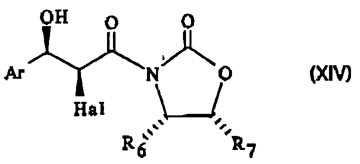 dans laquelle Ar est défini comme précédemment, Hal représente un atome d'halogène, de préférence un atome de brome, et R₆ et R₇, identiques ou différents, représentent un atome d'hydrogène ou un radical alcoyle contenant 1 à 4 atomes de carbone ou un radical phényle, l'un au moins étant un radical alcoyle ou un radical phényle, au moyen d'un alcoolate alcalin, éventuellement préparé in situ, dans un solvant organique inerte tel que le tétrahydrofuranne à une température comprise entre -80 et 25°C.

Le produit de formule générale (XTV) peut être obtenu par action d'un aldéhyde de formule générale :

Ar-CHO (XV)

dans laquelle Ar est défini comme précédemment sur un halogénure de formule générale : 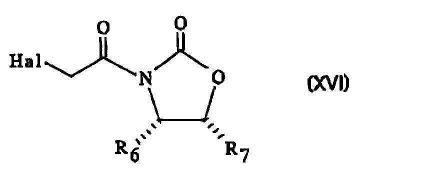 dans laquelle Hal, R₆ et R₇ sont définis comme précédemment, préalablement anionisé.

Généralement, on opère dans un solvant organique inerte choisi parmi les éthers (éther éthylique) et les hydrocarbures aliphatiques halogénés (chlorure de méthylène) à une température comprise entre -80 et 25°C, en présence d'une amine tertiaire (triéthylamine) et d'un agent d'énolisation (triflate de di-n.butylbore).

Le produit de formule générale (XVI) peut être obtenu par action d'un halogénure d'un acide halogénoacétique, de préférence le bromure de l'acide bromo-acétique, sur l'oxazolidinone correspondante.

Le produit de formule générale (XI) peut être obtenu par hydrogénolyse d'un produit de formule générale : 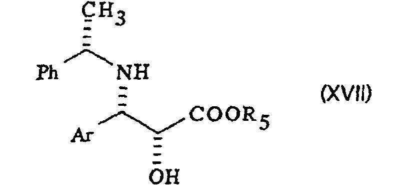 dans laquelle Ar et R₅ sont définis comme précédemment et Ph représente un radical phényle éventuellement substitué.

Généralement, l'hydrogénolyse est effectuée au moyen d'hydrogène en présence de catalyseur. Plus particulièrement, on utilise comme catalyseur un palladium sur charbon contenant 1 à 10 % en poids de palladium ou le dihydroxyde de palladium à 20 % en poids de palladium.

L'hydrogénolyse est effectuée dans un solvant organique ou dans un mélange de solvants organiques. Il est avantageux d'opérer dans l'acide acétique éventuellement associé à un alcool aliphatique contenant 1 à 4 atomes de carbone tel qu'un mélange acide acétique-méthanol à une température comprise entre 20 et 80°C.

L'hydrogène nécessaire à l'hydrogénolyse peut aussi être fourni par un composé qui libère de l'hydrogène par réaction chimique ou par décomposition thermique (formiate d'ammonium). Il est avantageux d'opérer sous une pression d'hydrogène comprise entre 1 et 50 bars.

Le produit de formule générale (XVII) peut être obtenu par hydrolyse ou alcoolyse d'un produit de formule générale : 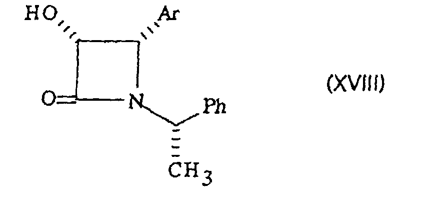 dans laquelle Ar et Ph sont définis comme précédemment.

Il est particulièrement avantageux d'effectuer une alcoolyse au moyen d'un alcool de formule R₅-OH dans laquelle R₅ est défini comme précédemment en opérant en milieu acide.

De préférence, on effectue l'alcoolyse au moyen de méthanol en présence d'un acide minéral fort tel que l'acide chlorhydrique à une température voisine de la température de reflux du mélange réactionnel.

Le produit de formule générale (XVIII) peut être obtenu par saponification d'un ester de formule générale : 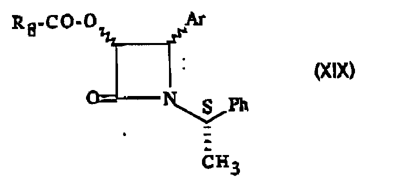 dans laquelle Ar et Ph sont définis comme précédemment et R₈ représente un radical alcoyle, phénylalcoyle ou phényle, suivie de la séparation du diastéréoisomère 3R,4S de formule générale (XVII) des autres diastéréoisomères.

Généralement, la saponification est effectuée au moyen d'une base minérale ou organique telle que l'ammoniaque, la lithine, la soude ou la potasse dans un solvant convenable tel qu'un mélange méthanol-eau ou tétrahydrofuranne-eau à une température comprise entre -10 et 20°C.

La séparation du diastéréoisomère 3R,4S peut être effectuée par cristallisation sélective dans un solvant organique convenable tel que l'acétate d'éthyle.

Le produit de formule générale (XIX) peut être obtenu par cycloaddition d'une imine de formule générale : 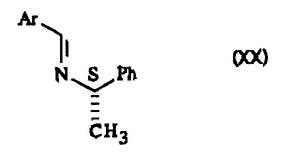 dans laquelle Ar et Ph sont définis comme précédemment, sur un halogénure d'acide de formule générale : 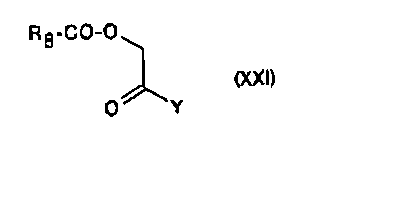 dans laquelle R₈ est défini comme précédemment et Y représente un atome d'halogène tel qu'un atome de brome ou de chlore.

Généralement la réaction est effectuée à une température comprise entre 0 et 50°C en présence d'une base choisie parmi les amines tertiaires aliphatiques (triéthylamine) ou la pyridine dans un solvant organique choisi parmi les hydrocarbures aliphatiques éventuellement halogénés (chlorure de méthylène, chloroforme) et les hydrocarbures aromatiques (benzène, toluène, xylènes).

Le produit de formule générale (XX) peut être obtenu dans les conditions analogues à celles décrites par M. Furukawa et coll., Chem. Pharm. Bull., 25 (1). 181-184 (1977).

Le produit de formule générale (VI) peut être obtenu par action d'un halogénure de métal alcalin (iodure de sodium, fluorure de potassium) ou d'un azoture de métal alcalin (azoture de sodium) ou un sel d'ammonium quaternaire ou un phosphate de métal alcalin sur un dérivé de la baccatine III ou de la désacétyl-10 baccatine III de formule générale: 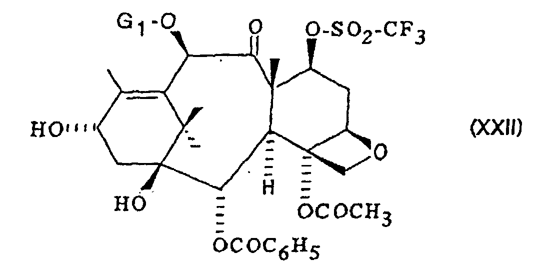 dans laquelle G₁ est défini comme précédemment.

Généralement la réaction est effectuée dans un solvant organique choisi parmi les éthers (tétrahydrofuranne, diisopropyléther, methyl t.butyléther) et les nitriles (acétonitrile) seul ou en mélange à une température comprise entre 20°C et la température d'ébullition du mélange réactionnel.

Le produit de formule (XXII) dans laquelle G₁ représente un atome d'hydrogène ou un radical acétyle, alcoxyacéthyle ou alcoyle peut être obtenu par action d'un dérivé de l'acide trifluorométhanesulfonique tel que l'anhydride ou le N-phényltrifluoro-méthanesulfonimide sur la baccatine III ou la désacétyl-10 baccatine III, qui peuvent être extraites selon les méthodes connues à partir des feuilles d'ifs (Taxus baccata), suivi éventuellement de la protection en position 10, étant entendu que pour obtenir un produit de formule générale (XXII) dans laquelle G₁ représente un radical alcoxyacétyle ou alcoyle, il est nécessaire de traiter préalablement la désacétyl-10 baccatine III protégée en -7, de préférence par un radical silylé, par un halogénure d'acide alcoxyacétique ou par un halogénure d'alcoyle.

Généralement, la réaction d'un dérivé de l'acide trifluorométhanesulfonique s'effectue dans un solvant organique inerte (hydrocarbures aliphatiques éventuellement halogènes, hydrocarbures aromatiques) en présence d'une base organique telle qu'une amine tertiaire aliphatique (triéthylamine) ou la pyridine à une température comprise entre -50 et +20°C.

Généralement l'introduction d'un groupement alcoxyacétyle s'effectue en traitant la désacétyl-10 baccatine III protégée par un halogénure d'acide alcoxyacétique en opérant dans un solvant organique basique tel que la pyridine à une température voisine de 20°C.

Généralement l'introduction d'un radical alcoyle s'effectue en traitant la désacétyl-10 baccatine III protégée et métallée en -10, au moyen par exemple d'un hydrure alcalin (hydrure de sodium) ou d'un alcoylure métallique (butyllithium), par un halogénure d'alcoyle.

2) par action d'un halogénure de métal alcalin (iodure de sodium, fluorure de potassium) ou d'un azoture de métal alcalin (azoture de sodium) ou un sel d'ammonium quaternaire ou un phosphate de métal alcalin sur un produit de formule générale : 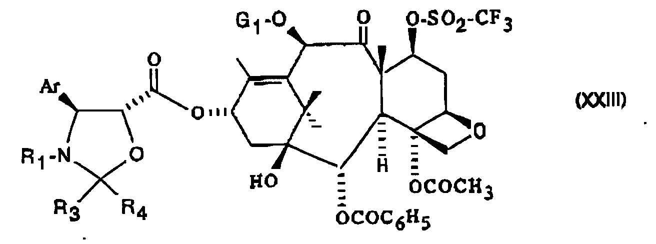 dans laquelle Ar, R₁, R₃, R₄ et G₁ sont définis comme précédemment.

Généralement, la réaction est effectuée dans un solvant organique choisi parmi les éthers (tétrahydrofuranne, diisopropyléther, méthyl t.butyléther) et les nitrites (acétonitrile) seul ou en mélange à une température comprise entre 20°C et la température d'ébullition du mélange réactionnel.

Le produit de formule générale (XXIII) peut être obtenu par action d'un dérivé de l'acide trifluorométhanesulfonique tel que l'anhydride ou le N-phényl trifluorométhanesulfonimide sur un taxoïde de formule générale : 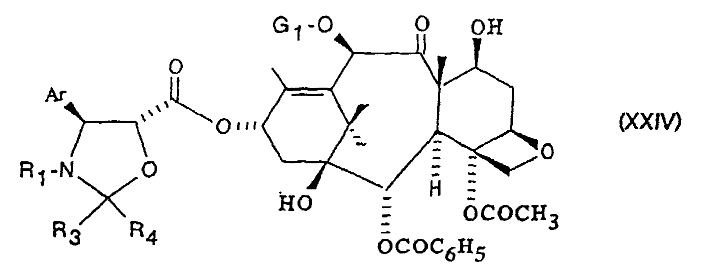 dans laquelle Ar, R₁, R₃, R₄ et G₁ sont définis comme précédemment.

Généralement, la réaction s'effectue dans un solvant organique inerte (hydrocarbures aliphatiques éventuellement halogénés, hydrocarbures aromatiques) en présence d'une base organique telle qu'une amine tertiaire aliphatique (triéthylamine) ou la pyridine à une température comprise entre -50 et +20°C.

Le taxoïde de formule générale' (XXIV), dans laquelle G₁ représente un atome d'hydrogène ou un radical acétyle, peut être obtenu à partir d'un produit de formule générale : 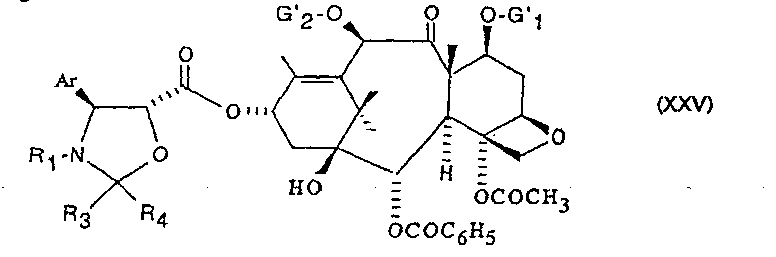 dans laquelle Ar, R₁, R₃ et R₄ sont définis comme précédemment, G'₁ représente un groupement protecteur de la fonction hydroxy et G'₂ représente un radical acétyle, alcoxyacétyle ou alcoyle ou un groupement protecteur de la fonction hydroxy par remplacement des groupements protecteurs G'₁ et éventuellement G'₂ par des atomes d'hydrogène,

Les radicaux G'₁ et G'₂, lorsqu'ils représentent un groupement protecteur de la fonction hydroxy, sont de préférence des radicaux trichloro-2,2,2 éthoxycarbonyle, (trichlorométhyl-2 propoxy)-2 carbonyle ou des radicaux trialkylsilyles, dialkylarylsilyles, alkyldiarylsilyles ou triarylsilyles dans lesquels les parties alkyles contiennent 1 à 4 atomes de carbone et les parties aryles sont de préférence des radicaux phényles, G'₂ pouvant en outre représenter un radical alcoxyacétyle.

Lorsque G'₁ et G'₂ représentent un radical trichloro-2,2,2 éthoxycarbonyle ou (trichlorométhyl-2 propoxy)-2 carbonyle, le remplacement des groupements protecteurs par des atomes d'hydrogène est effectué par le zinc, éventuellement associé au cuivre, en présence d'acide acétique à une température comprise entre 20 et 60°C ou au moyen d'un acide minéral ou organique tel que l'acide chlorhydrique ou l'acide acétique en solution dans un alcool aliphatique contenant 1 à 3 atomes de carbone ou un ester aliphatique tel que l'acétate d'éthyle, l'acétate d'isopropyle ou l'acétate de n.butyle en présence de zinc éventuellement associé à du cuivre.

Lorsque G'₁ représente un radical silylé et G'₂ représente un radical acétyle, alcoxyacétyle ou alcoyle, le remplacement du groupement protecteur G'₁ par un atome d'hydrogène peut s'effectuer au moyen, par exemple, d'acide chlorhydrique gazeux en solution éthanolique à une température voisine de 0°C, dans des conditions qui sont sans effet sur le reste de la molécule.

Lorsque G'₂ représente un radical alcoxyacétyle son remplacement éventuel par un atome d'hydrogène s'effectue par traitement en milieu alcalin ou par action d'un halogénure de zinc dans des conditions qui ne touchent pas au reste de la molécule. Généralement, le traitement alcalin est effectué par action de l'ammoniac en milieu hydro-alcoolique à une température voisine de 20°C. Généralement, le traitement par un halogénure. de zinc, de préférence l'iodure de zinc est effectué dans le méthanol à une température voisine de 20°C.

Le produit de formule générale (XXV) peut être obtenu dans les conditions décrites dans la demande internationale PCT/WO 9209589.

Les nouveaux dérivés de formule générale (I) peuvent aussi être obtenus par estérification d'un produit de formule générale (VI) au moyen d'un acide de formule générale : 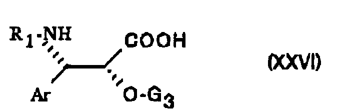 dans laquelle Ar et R₁ sont définis comme précédemment et G₃ représente un groupement protecteur de la fonction hydroxy choisi parmi les radicaux méthoxyméthyle, éthoxy-1 éthyle, benzyloxyméthyle, (β-triméthylsilyloxy)méthyle, tétrahydropyranyle, trichloro-2,2,2 éthoxyméthyle, trichloro-2,2,2 éthoxycarbonyle, (trichlorométhyl-2 propoxy)-2 carbonyle ou CH₂-Ph dans lequel Ph représente un radical phényle éventuellement substitué par un ou plusieurs atomes ou radicaux, identiques ou différents choisis parmi les atomes d'halogène et les radicaux alcoyles contenant 1 à 4 atomes de carbone ou alcoxy contenant 1 à 4 atomes de carbone, ou d'un dérivé activé de cet acide, pour obtenir un produit de formule générale : 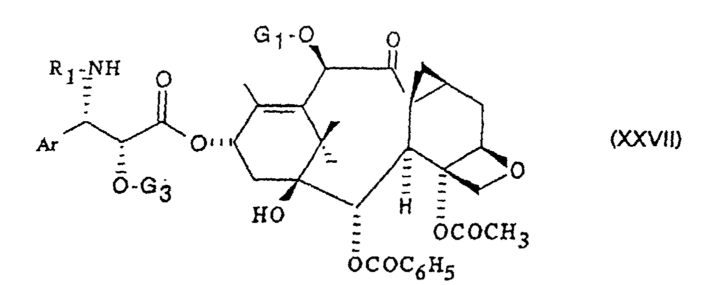 dans laquelle Ar, R_{1,} G₁ et G₃ sont définis comme précédemment, suivie du remplacement des groupements protecteurs G₁ et G₃ par des atomes d'hydrogène pour obtenir un produit de formule générale (I).

L'estérification peut être réalisée dans les conditions décrites précédemment pour l'estérification du produit de formula générale (VI) au moyen d'un acide de formule générale (VII).

Le remplacement des groupements protecteurs G₁ et G₃ du produit de formule générale (XXVII) par un atome d'hydrogène est effectué par traitement par le zinc, éventuellement associé au cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone ou un ester aliphatique tel que l'acétate d'éthyle, l'acétate-d'isopropyle ou l'acétate de n.butyle en présence de zinc éventuellement associé à du cuivre, lorsque G₁ et G₃ représentent un radical trichloro-2,2,2 éthoxycarbonyle ou (trichlorométhyl-2 propoxy)-2 carbonyle. Le remplacement du groupement protecteur G₃, lorsqu'il représente un radical silylé ou un reste d'acétal, peut être effectué par traitement en milieu acide tel que par exemple l'acide chlorhydrique en solution dans un alcool aliphatique contenant 1 à 3 atomes de carbone (méthanol, éthanol, propanol, isopropanol) ou l'acide fluorhydrique aqueux à une température comprise entre 0 et 40°C, lorsqu'il représente un reste d'acétal, le remplacement du groupement protecteur G₁ étant ensuite effectué dans les conditions décrites ci-dessus. Lorsque G₃ représente un groupement -CH₂-Ph, le remplacement de ce groupement protecteur par un atome d'hydrogène peut s'effectuer par hydrogénolyse en présence d'un catalyseur.

L'acide de formule générale (XXVI) peut être obtenu par saponification d'un ester de formule générale : 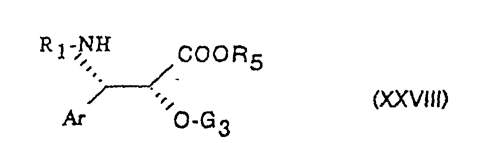 dans laquelle Ar, R₁, R₅ et G₃ sont définis comme précédemment.

Généralement la saponification est effectuée au moyen d'une base minérale (hydroxyde, carbonate ou bicarbonate de métal alcalin) en milieu hydro-alcoolique (méthanol-eau) à une température comprise entre 10 et 40°C.

L'ester de formule générale (XXVIII) peut être obtenu selon les méthodes habituelles de préparation des éthers, et plus particulièrement selon les procédés décrits par J-N. DENIS et coll., J. Org. Chem., 51, 46-50 (1986), à partir d'un produit de formule générale (XI).

Les nouveaux produits de formule générale (I) obtenus par la mise en oeuvre des procédés selon l'invention peuvent être purifiés selon les méthodes connues telles que la cristallisation ou la chromatographie.

Les produits de formule générale (I) présentent des propriétés biologiques remarquables.

In vitro, la mesure de l'activité biologique est effectuée sur la tubuline extraite de cerveau de porc par la méthode de M.L. Shelanski et coll., Proc. Natl. Acad. Sci. USA, 70, 765-768 (1973). L'étude de la dépolymérisation des microtubules en tubuline est effectuée selon la méthode de G. Chauvière et coll., C.R. Acad. Sci., 293, série II, 501-503 (1981). Dans cette étude les produits de formule générale (I) se sont montrés au moins aussi actifs que le taxol et le Taxotère.

In vivo, les produits de formule générale (I) se sont montrés actifs chez la souris greffée par le melanome B16 à des doses comprises entre 1 et 10 mg/kg par voie intrapéritonéale, ainsi que sur d'autres tumeurs liquides ou solides.

Les nouveaux produits ont des propriétés anti-tumorales et plus particulièrement une activité sur les tumeurs qui sont résistantes au Taxol® ou au Taxotère®. De telles tumeurs comprennent les tumeurs du colon qui ont une expression élevée du gène mdr 1 (gène de la multi-drug résistance). La multi-drug resistance est un terme habituel se rapportant à la résistance d'une tumeur à différents produits de structures et de mécanismes d'action différents. Les taxoïdes sont généralement connus pour être fortement reconnus par des tumeurs expérimentales telles que P388/DOX, une lignée cellulaire sélectionnée pour sa résistance à la doxorubicine (DOX) qui exprime mdr 1,

En particulier, il a été trouvé que les nouveaux produits de la présente invention comprenant les produits des exemples 1, 2 et 3 ont une multi-drug résistance meilleure que celle du Taxol® et du Taxotère®. De plus il a été trouvé de manière surprenante que le produit de l'exemple 3 a des propriétés de multi-drug résistance meilleure que celle des produits des exemples 1 et 2.

Les exemples suivants illustrent la présente invention.

### EXEMPLE 1

Une solution de 2,01 g de tert-butoxycarbonyl-3 diméthyl-2,2 phényl-4 oxazolidinecarboxylate-5-(4S,5R) d'acétoxy-4 benzoyloxy-2α epoxy-5β,20 dihydroxy-1β,10β méthylène-7β,8β oxo-9 nor-19 taxène-11 yle-13α dans 20 cm3 d'acide formique est agitée pendant 4 heures à une température voisine de 20°C puis concentrée à sec sous pression réduite (0,27 kPa) à 40°C. La meringue obtenue est dissoute dans 100 cm3 de dichlorométhane et la solution obtenue est additionnée de 20 cm3 d'une solution aqueuse saturée d'hydrogénocarbonate de sodium. La phase aqueuse est séparée par décantation et extraite par 20 cm3 de dichlorométhane. Les phases organiques sont réunies, séchées sur sulfate de magnésium, filtrées puis concentrées à sec sous pression réduite (2,7 kPa) à 40°C. On obtient 1,95 g d'une meringue blanche que l'on purifie par chromatographie sur 200 g de silice (0,063-0,2 mm) contenus dans une colonne de 7 cm de diamètre en éluant avec un mélange dichlorométhane-méthanol (98-2 en volumes) en recueillant des fractions de 30 cm3. Les fractions ne contenant que le produit cherché sont réunies et concentrées à sec sous pression réduite (0,27 kPa) à 40°C pendant 2 heures. On obtient ainsi 1,57 g d'amino-3 hydroxy-2 phényl-3 propionate-(2R,3S) d'acétoxy-4 benzoyloxy-2α époxy-5β,20 dihydroxy-1β,10β méthylène-7β,8β oxo-9 nor-19 taxène-11-yle-13α sous forme d'une meringue blanche.

A une solution de 400 mg d'amino-3 hydroxy-2 phényl-3 propionate-(2R,3S) d'acétoxy-4 benzoyloxy-2α époxy-5β,20 dihydroxy-1β,10β méthylène-7β,8β oxo-9 nor-19 taxène-11 yle-13α dans 1 cm3 de dichlorométhane, maintenue sous atmosphère d'argon, on ajoute 60 mg d'hydrogénocarbonate de sodium puis goutte à goutte, à une température voisine de 20°C, une solution de 0,16 g de dicarbonate de di.tert-butyle dans 1 cm3 de dichlorométhane. La solution obtenue est agitée pendant 64 heures à une température voisine de 20°C puis additionnée d'un mélange de 5 cm3 d'eau distillée et de 10 cm3 de dichlorométhane. La phase organique est lavée par 3 fois 2 cm3 d'eau distillée. La phase organique est séchée sur sulfate de magnésium, filtrée puis concentrée à sec sous pression réduite (2,7 kPa) à 40°C. On obtient ains 317 mg d'une meringue blanche que l'on purifie par chromatographie sur 30 g de silice (0,063-0,2 mm) contenus dans une colonne de 3 cm de diamètre en éluant avec un mélange dichlorométhane-méthanol (95-5 en volumes) en recueillant des fractions de 5 cm3. Les fractions ne contenant que le produit cherché sont réunies et concentrées à sec sous pression réduite (0,27 kPa) à 40°C pendant 2 heures. On obtient ainsi 161 mg de tert-butoxycarbonylamino-3 hydroxy-2 phényl-3 propionate-(2R,3S) d'acétoxy-4 benzoyloxy-2α époxy-5β,20 dihydroxy-1β,10β méthylène-7β,8β oxo-9 nor-19 taxène-11 yle-13α sous forme d'une meringue blanche dont les caractéristiques sont les suivantes :
- pouvoir rotatoire : [α]²⁰_{D} = - 17° (c = 0,482 ; méthanol)
- spectre de RMN du proton : (400 MHz ; CDCl₃ ; température de 323 K ; δ en ppm ; constantes de couplage J en Hz) : 1,21 (s, 3H : -CH₃ 16 ou 17) ; 1,28 (s, 3H : -CH₃ 16 ou 17) ; 1,34 [s, 9H : -C(CH₃)₃] ; de 1,30 à 1,50 (mt, 1H: -H 7) : 1,80 et 2,36 (2mt, 1H chacun : -CH₂- du cyclopropane) ; 1,88 (s, 3H : -CH₃ 18) ; 2,13 [mt, 1H : -(CH)-H 6] ; 2,26 [dd, 1H, J = 15 et 8,5 : -(CH)-H 14] ; 2,35 (s, 3H : -COCH₃) ; de 2,35 à 2,50 [mt, 2H : -(CH) -H 14 et -(CH)-H 6] ; 3,21 (d, 1H, J = 4 : -OH 2') ; 4,08 [d, 1H, J = 8 : -(CH)-H 20] ; 4,16 (d, 1H, J = 7 : -H 3) ; 4,18 (s, 1H, -OH 10) ; 4,31 [d, 1H, J = 8 : -(CH)-H 20] : 4,61 (dd, 1H, J = 4 et 2 : -H 2') ; 4,74 (d, 1H, J = 4 : -H 5) ; 5,00 (s, 1H : -H 10) ; 5,26 (dd, 1H, J = 9 et 2 : -H 3') ; 5,33 (d, 1H, J = 9 : -NH 3') ; 5,69 (d, 1H, J = 7 : -H 2) ; 6,29 (d, 1H, J = 8,5 : -H 13) ; de 7,30 à 7,50 [mt, 5H : -C₆H₅ en 3' (-H 2 à -H 6)] ; 7,51 [t, 2H, J = 7,5 : -OCOC₆H₅ (-H 3 et H 5)] ; 7,60 [t, 1H, J = 7,5 : -OCOC₆H₅ (-H 4)] ; 8,14 [d, 2H, J = 7,5 : -OCOC₆H₅ (-H 2 et H 6)].

Le tert-butoxycarbonyl-3 diméthyl-2,2 phényl-4 oxazolidinecarboxylate-5-(4S,5R) d'acétoxy-4 benzoyloxy-2α époxy-5β,20 dihydroxy-1β,10β méthylène-7β,8β oxo-9 nor-19 taxène-11 yle-13α peut être préparé de la manière suivante :

A une solution de 2,5 g de tert-butoxycarbonyl-3 diméthyl-2,2 phényl-4 oxazolidinecarboxylate-5-(4S,5R) d'acétoxy-4 benzoyloxy-2α époxy-5β,20 dihydroxy-1β,10β oxo-9 trifluorométhanesulfonate-7β taxène-11 yle-13α dans 25 cm3 d'acétonitrile anhydre et 3 cm3 de tétrahydrofuranne anhydre, maintenue sous atmosphère d'argon, on ajoute 2,5 g d'azoture de sodium. Le mélange réactionnel est chauffé pendant 2 heures sous agitation et sous atmosphère d'argon à une température voisine de 80°C, puis refroidi à une température voisine de 20°C et additionné de 30 cm3 d'eau distillée. La phase aqueuse est séparée par décantation puis extraite par 20 cm3 de dichlorométhane. Les phases organiques rassemblées sont séchées sur sulfate de magnésium, filtrées puis concentrées à sec sous pression réduite (2,7 kPa) à 40°C. On obtient ainsi 2,44 g d'une meringue jaune que l'on purifie par chromatographie sur 300 g de silice (0,063-0,2 mm) contenus dans une colonne de 8 cm de diamètre en éluant avec un mélange dichlorométhane-acétate d'éthyle (90-10 en volumes) en recueillant des fractions de 60 cm3. Les fractions 47 à 70 sont réunies et concentrées à sec sous pression réduite (0,27 kPa) à 40°C pendant 2 heures. On obtient ainsi 2,01 g de tert-butoxycarbonyl-3 diméthyl-2,2 phényl-4 oxazolidine-carboxylate-5-(4S,5R) d'acétoxy-4 benzoyloxy-2α époxy-5β,20 dihydroxy-1β,10β méthylène-7β,8β oxo-9 nor-19 taxène-11 yle-13α sous forme d'une meringue blanche.

Le tert-butoxycarbonyl-3 diméthyl-2,2 phényl-4 oxazolidinecarboxylate-5-(4S,5R) d'acétoxy-4 benzoyloxy-2α époxy-5β,20 dihydroxy-1β,10β oxo-9 trifluorométhanesulfonate-7β taxène-11 yle-13α peut être préparé de la manière suivante :

A une solution de 2,86 g de tert-butoxycarbonyl-3 diméthyl-2,2 phényl-4 oxazolidinecarboxylate-5-(4S,5R) d'acétoxy-4 benzoyloxy-2α époxy-5β,20 trihydroxy-1β,7β,10β oxo-9 taxène-11 yle-13α dans 29 cm3 de dichlorométhane anhydre, maintenue sous atmosphère d'argon, on ajoute 0,955 cm3 de pyridine et 50 mg de tamis moléculaire 4Å activé en poudre. Le mélange réactionnel est refroidi à une température voisine de -35°C, additionné lentement de 0,85 cm3 d'anhydride trifluorométhanesulfonique, agité à une température voisine de -5°C pendant 15 minutes et additionné de 10 cm3 d'eau distillée. Après filtration sur verre fritté garni de célite et rinçage du verre fritté par 3 fois 10 cm3 d'un mélange méthanol-dichlorométhane (10-90 en volumes), la phase aqueuse est séparée par décantation et extraite par 2 fois 10 cm3 de dichlorométhane. Les phases organiques sont réunies, séchées sur sulfate de magnésium, filtrées puis concentrées à sec sous pression réduite (2,7 kPa) à 40°C. On obtient 3,87 g d'une meringue blanche que l'on purifie par chromatographie sur 400 g de silice (0,063-0,2 mm) contenus dans une colonne de 10 cm de diamètre en éluant avec un gradient de dichlorométhane-acétate d'éthyle (de 97,5-2,5 à 90-10 en volumes) en recueillant des fractions de 80 cm3. Les fractions ne contenant que le produit cherché sont réunies et concentrées à sec sous pression réduite (0,27 kPa) à 40°C pendant 2 heures. On obtient ainsi 3,0 g de tert-butoxycarbonyl-3 diméthyl-2,2 phényl-4 oxazolidinecarboxylate-5-(4S,5R) d'acétoxy-4 benzoyloxy-2α époxy-5β,20 dihydroxy-1β,10β oxo-9 trifluorométhane-sulfonate-7β taxène-11 yle-13α sous forme d'une meringue blanche.

Le tert-butoxycarbonyl-3 diméthyl-2,2 phényl-4 oxazolidinecarboxylate-5-(4S,5R) d'acétoxy-4 benzoyloxy-2α époxy-5β,20 trihydroxy-1β,7β,10β oxo-9 taxène-11 yle-13α peut être préparé de la manière suivante :

Une solution de 24,35 g de tert-butoxycarbonyl-3 diméthyl-2,2 phényl-4 oxazolidinecarboxylate-5-(4S,5R) d'acétoxy-4 benzoyloxy-2α époxy-5β,20 oxo-9 bis-(trichloro-2,2,2 éthoxy)carbonyloxy-7β,10β hydroxy-1β taxène-11 yle-13α dans un mélange de 130 cm3 d'acétate d'éthyle et de 46,5 cm3 d'acide acétique est chauffée sous agitation et sous atmosphère d'argon jusqu'à une température voisine de 60°C puis additionnée de 40 g de zinc en poudre. Le mélange réactionnel est ensuite agité pendant 30 minutes à 60°C puis refroidi à une température voisine de 20°C et filtré sur verre fritté garni de célite. Le verre fritté est lavé par 100 cm3 d'un mélange méthanol-dichlorométhane (20-80 en volumes) ; les filtrats sont réunis puis concentrés à sec sous pression réduite (0,27 kPa) à une température voisine de 40°C.

Le résidu est additionné de 500 cm3 de dichlorométhane. La phase organique est lavée par 2 fois 50 cm3 d'une solution aqueuse saturée d'hydrogénocarbonate de sodium puis par 50 cm3 d'eau distillée. Les phases aqueuses obtenues par décantation et réunies sont extraites par 2 fois 30 cm3 de dichlorométhane. Les phases organiques sont réunies, séchées sur sulfate de magnésium, filtrées puis concentrées à sec sous pression réduite (2,7 kPa) à 40°C. On obtient 19,7 g d'une meringuè blanche que l'on purifie par chromatographie sur 800 g de silice (0,063-0,2 mm) contenus dans une colonne de 10 cm de diamètre en éluant avec un gradient dichlorométhane-méthanol (de 100-0 à 97-3 en volumes) en recueillant des fractions de 80 cm3. Les fractions ne contenant que le produit cherché sont réunies et concentrées à sec sous pression réduite (0,27 kPa) à 40°C pendant 2 heures. On obtient ainsi 16,53 g de tert-butoxycarbonyl-3 diméthyl-2,2-phényl-4-oxazolidine carboxylate-5-(4S,5R) d'acétoxy-4 benzoyloxy-2α époxy-5β,20 trihydroxy-1β,7β,10β oxo-9 taxène-11 yle-13α sous forme d'une meringue blanche.

Le tert-butoxycarbonyl-3 diméthyl-2,2 phényl-4 oxazolidinecarboxylate-5-(4S,5R) d'acétoxy-4 benzoyloxy-2α époxy-5β,20 oxo-9 bis-(trichloro-2,2,2 éthoxy) carbonyloxy-7β,10β hydroxy-1β taxène-11 yle-13α peut être préparé selon la méthode décrite dans la demande internationale PCT WO 9209589.

### EXEMPLE 2

Ne fait pas partie de l'invention.

A une solution de 550 mg d'amino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α dans 17,5 cm3 d'acétate d'éthyle, on ajoute 45 cm3 d'eau distillée, 45cm3 d'une solution aqueuse saturée en hydrogénocarbonate de sodium, puis goutte à goutte, à une température voisine de 20°C, 0,096 cm3 de chlorure de benzoyle. Le mélange obtenu est agité pendant 10 minutes à une température voisine de 20°C. Après décantation, la phase aqueuse est extraite avec 2 fois 30 cm3 d'acétate d'éthyle. Les phases organiques rassemblées sont séchées sur sulfate de magnésium, filtrées puis concentrées à sec sous pression réduite (2,7 kPa) à 40°C. On obtient ainsi 670 mg d'une meringue blanche que l'on purifie par chromatographie à pression atmosphérique sur 50 g de silice (0,063-0,2 mm) contenus dans une colonne de 2,5 cm de diamètre en éluant avec un mélange méthanol-dichlorométhane (1-99 puis 2,5-97,5 en volumes) en recueillant des fractions de 10 cm3. Les fractions ne contenant que le produit cherché sont réunies et concentrées à sec sous pression réduite (2,7 kPa) à 40°C. On obtient ainsi 610 mg d'une meringue blanche. Un échantillon de 300 mg est purifié par chromatographie préparative sur 12 plaques de silice en couche mince (Kieselgel 60F254, Merck ; épaisseur 0,25 mm) en éluant avec un mélange méthanol-dichlorométhane (3-97 en volumes). Après élution de la zone correspondant au produit principal par un mélange méthanol-dichlorométhane (10-90 en volumes) puis évaporation des solvants sous pression réduite (0,27 kPa) à une température voisine de 40°C, on obtient 155,2 mg de benzoylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α sous forme d'une meringue blanche, dont les caractéristiques sont les suivantes :
- pouvoir rotatoire : [α]²⁰_{D} = -30,5° (c = 0,491 ; méthanol)
- spectre de R.M.N. du proton : (300 MHz ; CDCl₃ ; δ en ppm, constantes de couplage J en Hz) : 1,27 (s, 3H : -CH₃ 16 ou 17) ; 1,30 (s, 3H : -CH₃ 16 ou 17) ; 1,40 (mt, 1H : -H 7) ; 1,62 et 2,25 (q et m, 1H chacun : CH₂ du cyclopropane) ; 1,85 (s, 3H : -CH₃ 18) ; 1,96 (s, 1H : -OH en 1) ; 2,05 et 2,48 (d et m, 1H chacun : -CH₂-en 6) ; 2,24 (s, 3H : -COCH₃ en 10) ; 2,28 et 2,50 (m, 1H chacun : -CH₂- en 14) ; 2,45 (s, 3H : -COCH₃ en 4) ; 3,52 (d, 1H : -OH en 2') ; 4,10 et 4,35 (d, 1H chacun : -CH₂- en 20) ; 4,11 (d, 1H : -H 3) ; 4,77 (d large, 1H : -H 5) ; 4,82 (dd, 1H : -H 2'); 5,70 (d, 1H : -H en 2) ; 5,84 (dd, 1H : -H 3') ; 6,30 (t large, 1H : -H 13) ; 6,36 (s, 1H : -H 10) ; 7,00 (d, 1H : -CONH-) ; de 7,35 à 8,30 (m, 15H : -C₆H₅ en 3', -OCOC₆H₅ et NHCOC₆H₅).

L'amino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α peut être préparé en opérant dans les conditions décrites dans l'exemple 1 pour la préparation de l'amino-3 hydroxy-2 phényl-3 propionate-(2R,3S) d'acétaxy-4α benzoyloxy-2α époxy-5β,20 dihydroxy-1β,10β méthylène-7β,8β oxo-9 nor-19 taxène-11 yle-13α. Ainsi, à partir de 1,6 g de tert-butoxycarbonyl-3 diméthyl-2,2 phényl-4 oxazolidinecarboxylate-5-(4S,5R) de diacétoxy-4α,10β benzoyloxy-2α époxy-5β,20 hydroxy-1β méthylène-7β,8β oxo-9 nor-19 taxène-11 yle-13α, on obtient 1,14 g d'amino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α sous forme d'une meringue blanche.

Le tert-butoxycarbonyl-3 diméthyl-2,2 phényl-4 oxazolidinecarboxylate-5-(4S,5R) de diacétoxy-4α,10β benzoyloxy-2α époxy-5β,20 hydroxy-1β méthylène-7β,8β oxo-9 nor-19 taxène-11 yle-13 peut être préparé dans les conditions décrites dans l'exemple 1 pour la préparation du tert-butoxycarbonyl-3 diméthyl-2,2 phényl-4 oxazolidinecarboxylate-5-(4S,5R) d'acétoxy-4α benzoyloxy-2α époxy-5β,20 dihydroxy-1β,10β méthylène-7β,8β oxo-9 nor-19 taxène-11 yle-13α. Ainsi, à partir de 2,2 g de tert-butoxycarbonyl-3 diméthyl-2,2 phényl-4 oxazolidinecarboxylate-5-(4S,5R) de diacétoxy-4α,10β benzoyloxy-2α époxy-5β,20 hydroxy-1β oxo-9 trifluorométhanesulfonate-7β taxène-11 yle-13α, on obtient 1,62 g de tert-butoxycarbonyl-3 diméthyl-2,2 phényl-4 oxazolidinecarboxylate-5-(4S,5R) de diacétoxy-4α,10β benzoyloxy-2α époxy-5β,20 hydroxy-1β méthylène-7β,8β oxo-9 nor-19 taxène-11 yle-13 sous forme d'une meringue blanche.

Le tert-butoxycarbonyl-3 diméthyl-2,2 phényl-4 oxazolidinecarboxylate-5-(4S,5R) de diacétoxy-4α,10β benzoyloxy-2α époxy-5β,20 hydroxy-1β oxo-9 trifluorométhanesulfonate-7β taxène-11 yle-13α peut être dans les conditions décrites dans l'exemple 1 pour la préparation du tert-butoxycarbonyl-3 diméthyl-2,2 phényl-4 oxazolidinecarboxylate-5-(4S,5R) d'acétoxy-4α benzoyloxy-2α époxy-5β,20 dihydroxy-1β,10β oxo-9 trifluorométhanesulfonate-7β nor-19 taxène-11 yle-13α. Ainsi, à partir de 2,4 g de tert-butoxycarbonyl-3 diméthyl-2,2 phényl-4 oxazolidinecarboxylate-5-(4S,5R) de diacétoxy-4α,10β benzoyloxy-2α époxy-5β,20 dihydroxy-1β,7β oxo-9 taxène-11 yle-13α, on obtient 2,46 g de tert-butoxycarbonyl-3 diméthyl-2,2 phényl-4 oxazolidinecarboxylate-5-(4S,5R) de diacétoxy-4α,10β benzoyloxy-2α époxy-5β,20 hydroxy-1β oxo-9 trifluorométhanesulfonate-7β taxène-11 yle-13α sous forme d'une meringue blanche.

Le tert-butoxycarbonyl-3 diméthyl-2,2 phényl-4 oxazolidinecarboxylate-5-(4S,5R) de diacétoxy-4α,10β benzoyloxy-2α époxy-5β,20 dihydroxy-1β,7β oxo-9 taxène-11 yle-13α peut être préparé dans les conditions décrites dans la demande international PCT WO 9209589.

### EXEMPLE 3

A une solution de 550 mg d'amino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α dans 1 cm3 de dichlorométhane, maintenue sous atmosphère d'argon, on ajouté 76 mg d'hydrogénocarbonate de sodium puis goutte à goutte, à une température voisine de 20°C, une solution de 197 mg de dicarbonate de di-tert-butyle dans 1 cm3 de dichlorométhane. La solution obtenue est agitée pendant 15 heures à une température voisine de 20°C puis additionnée d'un mélange de 5 cm3 d'eau distillée et de 10 cm3 de dichlorométhane. La phase aqueuse est extraite par 5 cm3 de dichlorométhane. Les phases organiques rassemblées sont séchées sur sulfate de magnésium, filtrées puis concentrées à sec sous pression réduite (2,7 kPa) à 40°C. On obtient ainsi 780 mg d'une meringue blanche que l'on purifie par chromatographie à pression atmosphérique sur 50 g de silice (0,063-0,2 mm) contenus dans une colonne de 2,5 cm de diamètre en éluant avec un mélange méthanol-dichlorométhane (1-99 puis 2,5-97,5 en volumes) en recueillant des fractions de 10 cm3. Les fractions ne contenant que le produit cherché sont réunies et concentrées à sec sous pression réduite (2,7 kPa) à 40°C. On obtient ainsi 660 mg d'une meringue blanche. Un échantillon de 300 mg est purifié par chromatographie préparative sur 12 plaques de silice en couche mince (Kieselgel 60F254, Merck ; épaisseur 0,25 mm) en éluant avec un mélange méthanol-dichiorométhane (4-96 en volumes). Après élution de la zone correspondant au produit principal par un mélange méthanol-dichlorométhane (10-90 en volumes) puis évaporation des solvants sous pression réduite (0,27 kPa) à une température voisine de 40°C, on obtient 159,7 mg de tert-butoxycarbonyl-amino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α sous forme d'une meringue blanche, dont les caractéristiques sont les suivantes :
- pouvoir rotatoire : [α]²⁰_{D} = -34° (c = 0,564 ; méthanol)
- spectre de R.M.N. du proton : (400 MHz ; CDCl₃ ; δ en ppm ; constantes de couplage J en Hz) : 1,28 (s, 3H : -CH₃ 16 ou 17) ; 1,30 [s, 9H : -C(CH₃)₃] ; 1,38 (mt, 1H : -H 7) ; 1,60 (s, 3H : -CH₃ 16 ou 17) ; 1,68 et 2,25 (t et m, 1H chacun : CH₂ du cyclopropane) ; 1,85 (s, 3H: -CH₃ 18) ; 2,10 et 2,45 (d et td, 1H chacun : -CH₂- en 6) ; 2,23 (s, 3H: -COCH₃ en 10) ; 2,22 et 2,40 (m, 1H chacun: -CH₂- en 14) ; 2,40 (s, 3H : -COCH₃ en 4) ; 3,28 (d, 1H: -OH en 2') ; 4,05 et 4,22 (d, 1H chacun : -CH₂- en 20) ; 4,10 (d, 1H: -H 3) ; 4,62 (s large, 1H: -H 2') ; 4,73 (d, 1H : -H 5) ; 5,29 (d large, 1H: -H 3') ; 5,37 (d, 1H : -CONH-) ; 5,67 (d, 1H : -H en 2) ; 6,28 (t large, 1H : -H 13) ; 6,33 (s, 1H : -H 10) ; de 7,30 à 7,45 (mt, 5H : -C₆H₅ en 3') ; 7,51 [t, 2H : -OCOC₆H₅ (-H 3 et -H 5)] ; 7,61 [t, 1H : -OCOC₆H₅ (-H 4)] ; 8,17 [d, 2H : -OCOC₆H₅ (-H 2 et -H 6)],

### EXEMPLE 4

A une solution de 100 mg de désacétyl-10 baccatine III dans un mélange de 2 cm3 de tétrahydrofuranna et de 0,05 cm3 de pyridine, refroidie à une température voisine de -78°C et maintenue sous atmosphère d'argon, on ajoute, goutte à goutte, 0,09 cm3 d'anhydride trifluorométhane-sulfonique. On laisse la température remonter lentement à une température voisine de 0°C en une heure environ, puis jusqu'à une température voisine de 20°C en une heure environ. Aorès 2 heures à une température voisine de 20°C, on ajoute 200 mg d'iodure de tétrabutylammonium, puis la solution est chauffée à la température d'ébullition du solvant pendant 15 heures. Après refroidissement à une température voisine de 20°C, on ajoute 10 cm3 d'acétate d'éthyle puis 1 cm3 d'eau distillée. Après décantation, la phase organique est séchée sur sulfate de magnésium, filtrée et concentrée à sec sous pression réduite (2,7 kPa) à 40°C. On obtient ainsi 116 mg d'huile jaune qui est purifiée par chromatographie à pression atmosphérique sur 30 g de silice (0,063-0,2 mm) contenus dans une colonne de 2,5 cm de diamètre en éluant avec un mélange acétate d'éthyle-dichlorométhane avec un gradient d'élution de 0-100 à 80-20 en volumes. Les fractions contenant le produit recherché sont réunies et concentrées à sec sous pression réduite (0,27 kPa) à 40°C. On obtient ainsi 10,3 mg de désacétyl-10 méthylène-7β,8β nor-19 baccatine III sous forme d'une meringue blanche dont les caractéristiques sont les suivantes :
- spectre de R.M.N. du proton : (400 MHz ; CDCl₃ ; δ enppm ; constantes de couplage J en Hz) : 1,14 (s, 3H : -CH₃ en 16 ou 17) ; 1,42 (mt, 1H : -H en 7) ; 1,76 et 2,31 (t et m, 1H chacun : CH₂ du cyclopropane) ; 2,07 (s, 3H : -CH₃- en 18) ; 2,15 et 2,50 (d, large et td, 1H chacun : -CH₂ en 6) ; 2,30 (s, 3H : -COCH₃ en 4) ; 2,28 et 2,35 (m, 1H chacun : -CH₂ en 14) ; 4,11 et 4,37 (d, 1H chacun : -CH₂ en 20) ; 4,28 (d, 1H : -H en 3) ; 4,79 (d, 1H : -H en 5) ; 4,88 (t large, 1H : -H en 13) ; 5,09 (s, 1H: -H en 10) ; 5,66 (d, 1H : -H en 2) ; 7,51 [t, 2H : -OCOC₆H₅ (-H en 3 et 5)] ; 7,61 [t, 1H: -OCOC₆H₅ (-H en 4)] ; 8,17 [d, 2H : -OCOC₆H₅ (-H en 2 et 6)].
- spectre de R.M.N. du ¹³C : (100 MHz ; CDCl₃ ; δ en ppm ; non découplé ; s = singulet ; d = doublet ; t = triplet ; q = quadruplet) : 15 (q, C18) ; 16,5 (t, C19) ; 20 et 27 (q, C16 et C17) ; 22,5 (q, -COCH₃) ; 26,5 (t, C6) ; 33 (d, C7) ; 35 (s, C8) ; 39 (d, C3) ; 39,5 ( t, C14) ; 43 (s, C15) ; 68 (d, C13) ; 76 (t, C20) ; 76,2 (d, C10) ; 79,5 (s, C1) ; 80 (s, C4) ; 81 (d, C2) ; 85 (d, C5) ; 129 (d, C2 : -OCOC₆H₅) ; 130 (s, C1 de -OCOC₆H₅) ; 130,5 (d, C3 de -OCOC6H5) ; 134 (d, C4 de -OCOC₆H₅) ; 136 (s, C11) ; 143 (s, C12) ; 168 (s, -OCOC6H5) ; 171 (s, -COCH₃) ; 210 (s, C9).

Les nouveaux produits de formule générale (I) manifestent une activité inhibitrice significative de la prolifération cellulaire anormale et possèdent des propriétés thérapeutiques permettant le traitement de malades ayant des conditions pathologiques associées à une prolifération cellulaire anormale. Les conditions pathologiques incluent la prolifération cellulaire anormale de cellules malignes ou non malignes de divers tissus et/ou organes, comprenant, de manière non limitative, les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales. Ces conditions pathologiques peuvent inclure également le psoriasis, les tumeurs solides, les cancers de l'ovaire, du sein, du cerveau, de la prostate, du colon, de l'estomac, du rein ou des testicules, le sarcome de Kaposi, le cholangiocarcinome, le choriocarcinome, le neuroblastome, la tumeur de Wilms, la maladie de Hodgkin, les mélanomes, les myélomes multiples, les leucémies lymphocytaires chroniques; les lymphomes granulocytaires aigus ou chroniques. Les nouveaux produits selon l'invention sont particulièrement utiles pour le traitement du cancer de l'ovaire. Les produits selon l'invention peuvent être utilisés pour prévenir ou retarder l'apparition ou la réapparition des conditions pathologiques ou pour traiter ces conditions pathologiques.

Les produits selon l'invention peuvent être administrés à un malade selon différentes formes adaptées à la voie d'administration choisie qui, de préférence, est la voie parentérale. L'administration par voie parentérale comprend les administrations intraveineuse, intrapéritonéale, intramusculaire ou sous-cutanée. Plus particulièrement préférée est l'administration intrapéritonéale ou intraveineuse.

La présente invention comprend également les compositions pharmaceutiques qui contiennent au moins un produit de formule générale (I) en une quantité suffisante adaptée à l'emploi en thérapeutique humaine ou vétérinaire. Les compositions peuvent être préparées selon les méthodes habituelles en utilisant un ou plusieurs adjuvants, supports ou excipients pharmaceutiquement acceptables. Les supports convenables incluent les diluants, les milieux aqueux stériles et divers solvants non toxiques. De préférence les compositions se présentent sous forme de solutions ou de suspensions aqueuses, de solutions injectables qui peuvent contenir des agents émusifiants, des colorants, des préservatifs ou des stabilisants.

Le choix des adjuvants ou excipients peut être déterminé par la solubilité et les propriétés chimiques du produit, le mode particulier d'administration et les bonnes pratiques pharmaceutiques.

Pour l'administration parentérale, on utilise des solutions ou des suspensions stériles aqueuses ou non aqueuses. Pour la préparation de solutions ou de suspensions non aqueuses peuvent être utilisés des huiles végétales naturelles telle que l'huile d'olive, l'huile de sésame ou l'huile de paraffine ou les esters organiques injectables tel que l'oléate d'éthyle. Les solutions stériles aqueuses peuvent être constituées d'une solution d'un sel pharmaceutiquement acceptable en solution dans de l'eau. Les solutions aqueuses conviennent pour l'administration intraveineuse dans la mesure où le pH est convenablement ajusté et où l'isotonicité est réalisée, par exemple, par une quantité suffisante de chlorure de sodium ou de glucose. La stérilisation peut être réalisée par chauffage ou par tout autre moyen qui n'altère pas la composition.

Il est bien entendu que tous les produits entrant dans les compositions selon l'invention doivent être purs et non toxiques pour les quantités utilisées.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Le traitement thérapeutique peut être effectué concuremment avec d'autres traitements thérapeutiques incluant des médicaments antinéoplastiques, des anticorps monoclonaux, des thérapies immunologiques ou des radiothérapies ou des modificateurs des réponses biologiques. Les modificateurs des réponses incluent, de manière non limitative, les lymphokines et les cytokines telles que les interleukines, les interférons (α, β ou δ) et le TNF. D'autres agents chimiothérapeutiques utiles dans le traitement des désordres dus à la prolifération anormale des cellules incluent, de manière non limitative, les agents alkylants tels que les moutardes à l'azote comme la mechloretamine, le cyclophosphamide, le melphalan et le chlorambucil, des sulfonates d'alkyle comme le busulfan, les nitrosourées comme la carmmstine, la lomustine, la sémustine et la streptozocine, les triazènes comme la dacarbazine, les antimétabolites comme les analogues de l'acide folique tel que le méthotrexate, les analogues de pyrimidine comme le fluorouracil et la cytarabine, des analogues de purines comme la mercaptopurine et la thioguanine, des produits naturels tels que les alcaloïdes de vinca comme la vinblastine, la vincristine et la vendésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination du platine tel que le cisplatine, les urées substituées tel que l'hydroxyurée, les dérivés de méthylhydrazine comme la procarbazine, les suppresseurs adrénocoticoïques comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynylestradiol, les antioestrogène comme le tamoxifène, les androgènes comme le propionate de testostérone et la fluoxymesterone.

Les doses utilisées pour mettre en oeuvre les méthodes selon l'invention sont celles qui permettent un traitement prophylactique ou un maximum de réponse thérapeutique. Les doses variant selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale. Les produits selon l'invention peuvent être administrés aussi souvent que nécessaire pour obtenir l'effet thérapeutique désiré. Certains malades peuvent répondre rapidement à des doses relativement fortes ou faibles puis avoir besoin de doses d'entretien faibles ou nulles. Généralement, de faibles doses seront utilisées au début du traitement et, si nécessaire, des doses de plus en plus fortes seront administrées jusqu'à l'obtention d'un effet optimum. Pour d'autres malades il peut être nécessaire d'administrer des doses d'entretien 1 à 8 fois par jour, de préférence 1 à 4 fois, selon les besoins physiologiques du malade considéré. Il est aussi possible que pour certains malades il soit nécessaire de n'utiliser qu'une à deux administrations journalières.

Chez l'homme, les doses sont généralement comprises entre 0,01 et 200 mg/kg. Par voie intrapéritonéale, les doses seront en général comprises entre 0,1 et 100 mg/kg et, de préférence entre 0,5 et 50 mg/kg et, encore plus spécifiquement entre 1 et 10 mg/kg. Par voie intraveineuse, les doses sont généralement comprises entre 0,1 et 50 mg/kg et, de préférence entre 0,1 et 5 mg/kg et, encore plus spécifiquement entre 1 et 2 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

L'exemple suivant illustre une composition selon l'invention.

### EXEMPLE

On dissout 40 mg du produit obtenu à l'exemple 1 dans 1 cm3 d'Emulphor EL 620 et 1 cm3 d'éthanol puis la solution est diluée par addition de 18 cm3 de sérum physiologique.

La composition est administrée par perfusion pendant 1 heure par introduction dans du soluté physiologique.

## Revendications

1. Taxoïdes de formule générale : 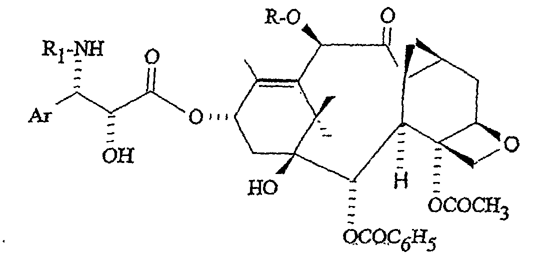 dans laquelle
R représente un atome d'hydrogène ou un radical acétyle, alcoxyacétyle ou alcoyle,
R₁ représente un radical benzoyle où un radical R₂-O-CO- dans lequel R₂ représente un radical alcoyle, alcényle, alcynyle, cycloalcoyle, cycîoalcényle, bicycloalcoyle, phényle ou hétérocyclyle éventuellement substitué, et
Ar représente un radical aryle éventuellement substitué.

2. Dérivés selon la revendication 1 pour lesquels :
R représente un atome d'hydrogène ou un radical acétyle, alcoxyacétyle ou alcoyle,
R₁ représente un radical benzoyle ou un radical R₂-O-CO dans lequel R₂ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants, identiques ou différents, choisis parmi les atomes d'halogène et les radicaux hydroxy, alcoxy contenant 1 à 4 atomes de carbone, dialcoylamino dont chaque partie alcoyle contient 1 à 4 atomes de carbone, pzpéridino, moipholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoxycarbonyle dont la partie alcoyle contient 1 à 4 atomes de carbone,
- ou un radical phényle, éventuellement substitué par un ou plusieurs radicaux, identiques ou différents, choisis parmi les radicaux alcoyles contenant 1 à 4 atomes de carbone ou alcoxy contenant 1 à 4 atomes de carbone,
- ou un radical hétérocyclyle azoté saturé ou non saturé contenant 5 ou 6 chaînons éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Ar représente un radical phényle ou a- ou b-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoyl-carbamoyle, cyano, nitro et trifluorométhyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou a- ou b-naphtyles, ou bien Ar représente un radical hétérocyclique aromatique ayant 5 chaînons et contenant un ou plusieurs atomes, identiques ou différents, choisis parmi les atomes d'azote, d'oxygène ou de soufre, éventuellement substitué par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

3. Dérivés selon la revendication 1 pour lesquels R représente un atome d'hydrogène ou un radical acétyle, alcoxyacétyle ou alcoyle, R₁ représente un radical benzoyle ou un radical R₂-O-CO- dans lequel R₂ représente un radical t.butyle et Ar représente un radical phényle.

4. Dérivé selon la revendication 1 pour lequel R représente un radical acétyle, R₁ représente un radical R₂-O-CO- dans lequel R₂ représente un radical t.butyle et Ar représente un radical phényle.

5. Dérivé selon la revendication 1 pour lequel R représente un atome d'hydrogène, R₁ représente un radical R₂-O-CO- dans lequel R₂ représente un radical t.butyle et Ar représente un radical phényle.

6. Procédé de préparation d'un produit selon l'une des revendications 1 à 5 **caractérisé en ce que** l'on estérifie un produit de formule générale : 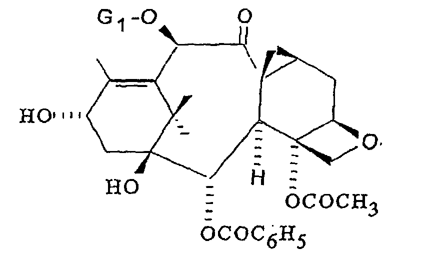 dans laquelle G₁ représente un atome d'hydrogène ou un radical acétyle, alcoxyacétyle ou alcoyle ou un groupement protecteur de la fonction hydroxy, au moyen d'un acide de formule générale : 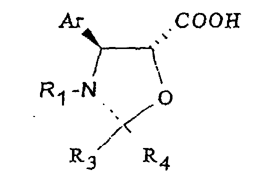 dans laquelle Ar et R₁ sont définis comme dans l'une des revendications 1 à 5, R₃ représente un atome d'hydrogène ou un radical alcoxy contenant 1 à 4 atomes de carbone ou un radical aryle éventuellement substitué et R₄ représente un atome d'hydrogène, pour obtenir un produit de formule générale : 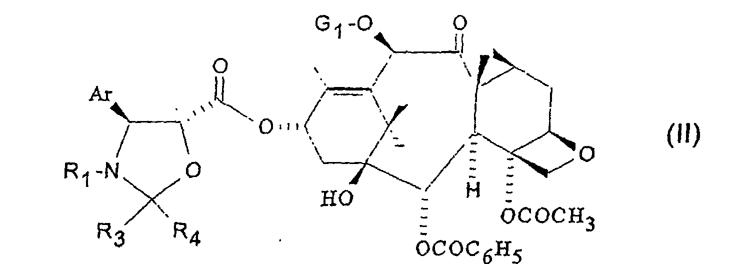 dans laquelle Ar, R et R₁ sont définis comme dans l'une des revendications 1 à 5, R₃, R₄ et G₁ sont définis comme ci-dessus, que l'on traite en milieu acide pour obtenir un produit de formule générale : 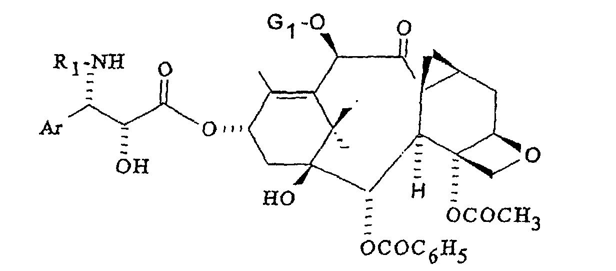 dans laquelle Ar, R₁ et G₁ sont définis comme ci-dessus, puis remplace éventuellement le groupement protecteur G₁ par un atome d'hydrogène et isole le produit obtenu.

7. Procédé selon la revendication 6 **caractérisé en ce que** l'estérification est effectuée au moyen de l'acide libre en opérant en présence d'un agent de condensation choisi parmi les carbodiimides et les carbonates réactifs et d'un agent d'activation choisi parmi les aminopyridines dans un solvant organique choisi parmi les éthers, les cétones, les esters, les nitriles, les hydrocarbures aliphatiques, les hydrocarbures aliphatiques halogénés et les hydrocarbures aromatiques à une température comprise entre -10 et 90°C.

8. Procédé selon la revendication 6 **caractérisé en ce que** l'estérification au moyen de l'anhydride est effectué en présence d'un agent d'activation choisi parmi les aminopyridines dans un solvant organique choisi parmi les éthers, les esters, les cétones, les nitriles, les hydrocarbures aliphatiques, les hydrocarbures aliphatiques halogénés et les hydrocarbures aromatiques à une température comprise entre 0 et 90°C.

9. Procédé selon la revendication 6 **caractérisé en ce que** l'estérification est effectuée au moyen d'un halogénure ou d'un anhydride avec un acide aliphatique ou aromatique, éventuellement préparé in situ, en opérant en présence d'une base choisie parmi les amines aliphatiques tertiaires dans un solvant organique choisi parmi les éthers, les esters, les cétones, les nitriles, les hydrocarbures aliphatiques, les hydrocarbures aliphatiques halogénés et les hydrocarbures aromatiques à une température comprise entre 0 et 80°C.

10. Procédé selon la revendication 6 **caractérisé en ce que** le traitement acide est effectué au moyen d'un acide minéral ou organique dans un solvant organique à une température comprise entre -10 et 60°C.

11. Procédé selon la revendication 10 **caractérisé en ce que** l'acide est choisi parmi les acides chlorhydrique, sulfutique, acétique, méthanesulfonique, trifluorométhanesulfonique et p.toluènesulfonique utilisés seuls ou en mélange.

12. Procédé selon la revendication 10 **caractérisé en ce que** le solvant est choisi parmi les alcools, les éthers, les esters, les hydrocarbures aliphatiques halogénés, les hydrocarbures aromatiques et les nitrites.

13. Procédé selon la revendication 6 **caractérisé en ce que** le remplacement par un atome d'hydrogène du groupement protecteur G₁, lorsqu'il représente un radical trichloro-2,2,2 éthoxycarbonyle ou (trichlorométhyl-2 propoxy)-2 carbonyle est effectué par traitement par le zinc, éventuellement associé au cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone ou un ester aliphatique tel que l'acétate d'éthyle, l'acétate d'isopropyle ou l'acétate de n.butyle en présence de zinc éventuellement associé à du cuivre, et, lorsqu'il représente un radical alcoxyacétyle, par traitement en milieu alcalin au moyen d'ammoniac en milieu hydro-alcoolique à une température voisine de 20°C ou par traitement par un halogénure de zinc dans le méthanol à une température voisine de 20°C.

14. Procédé de préparation d'un produit selon l'une des revendications 1 à 5 **caractérisé en ce que** l'on estérifie un produit de formule générale : 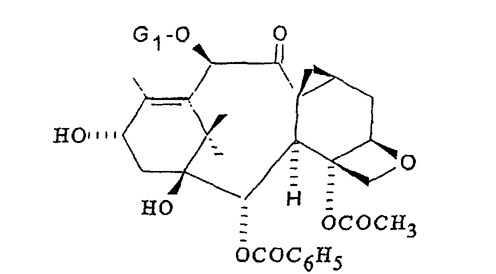 dans laquelle G₁ représente un atome d'hydrogène ou un radical acétyle, alcoxyacétyle ou alcoyle ou un groupement protecteur de la fonction hydroxy au moyen d'un acide de formule générale : 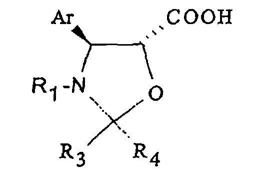 dans laquelle Ar et R₁ sont définis comme dans l'une des revendications 1 à 5 et R₃ et R₄, identiques ou différents, représentent un radical alcoyle contenant 1 à 4 atomes de carbone ou un radical aralcoyle dont la partie alcoyle contient 1 à 4 atomes de carbone ou un radical aryle, ou bien R₃ représente un radical trihalométhyle ou un radical phényle substitué par un radical trihalométhyle et R₄ représente un atome d'hydrogène, ou bien R₃ et R₄ forment ensemble avec l'atome de carbone auquel ils sont liés un cycle ayant 4 à 7 chaînons, pour obtenir après traitement en milieu acide un produit de formule générale : 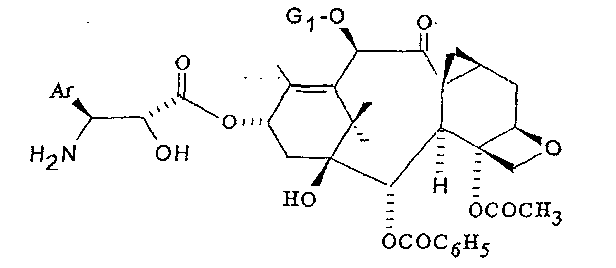 dans laquelle Ar est défini comme dans l'une des revendications 1 à 5 et G₁ est défini comme ci-dessus, qui est acylé au moyen de chlorure de benzoyle ou d'un dérivé réactif de formule générale :
R₂-O-CO-X
dans laquelle R₂ est défini comme dans l'une des revendications 1 à 5 et X représente un atome d'halogène ou un reste-O-R₂ ou -O-CO-O-R₂, puis remplace, si nécessaire, le groupement protecteur G₁ par un atome d'hydrogène, et isole le produit obtenu.

15. Procédé selon la revendication 14 **caractérisé en ce que** l'estérification est effectuée au moyen de l'acide libre en opérant en présence d'un agent de condensation choisi parmi les carbodiimides et les carbonates réactifs et d'un agent d'activation choisi parmi les aminopyridines dans un solvant organique choisi parmi les éthers, les cétones, les esters, les nitriles, les hydrocarbures aliphatiques, les hydrocarbures aliphatiques halogénés et les hydrocarbures aromatiques à une température comprise entre -10 et 90°C.

16. Procédé selon la revendication 14 **caractérisé en ce que** l'estérification au moyen de l'anhydride est effectué en présence d'un agent d'activation choisi parmi les aminopyridines dans un solvant organique choisi parmi les éthers, les esters, les cétones, les nitriles, les hydrocarbures aliphatiques, les hydrocarbures aliphatiques halogénés et les hydrocarbures aromatiques à une température comprise entre 0 et 90°C.

17. Procédé selon la revendication 14 **caractérisé en ce que** l'estérification est effectuée au moyen d'un halogénure ou d'un anhydride avec un acide aliphatique ou aromatique, éventuellement préparé in situ, en opérant en présence d'une base choisie parmi les amines aliphatiques tertiaires dans un solvant organique choisi parmi les éthers, les esters, les cétones, les nitriles, les hydrocarbures aliphatiques, les hydrocarbures aliphatiques halogènes et les hydrocarbures aromatiques à une température comprise entre 0 et 80°C.

18. Procédé selon la revendication 14 **caractérisé en ce que** le traitement acide est effectué au moyen d'un acide minéral ou organique dans un solvant organique à une température comprise entre 0 et 50°C.

19. Procédé selon la revendication 18 **caractérisé en ce que** l'acide est choisi parmi les acides chlorhydrique, sulfurique et formique.

20. Procédé selon la revendication 18 **caractérisé en ce que** le solvant est choisi parmi les alcools contenant 1 à 3 atomes de carbone.

21. Procédé selon la revendication 14 **caractérisé en ce que** l'acylation est effectuée dans un solvant organique inerte en présence d'une base minérale ou organique.

22. Procédé selon la revendication 21 **caractérisé en ce que** le solvant organique inerte est choisi parmi les esters et les hydrocarbures aliphatiques halogénés.

23. Procédé selon l'une des revendications 20, 21 ou 22 **caractérisé en ce que** l'on opère à une température comprise entre 0 et 50°C.

24. Procédé selon la revendication 14 **caractérisé en ce que** le remplacement par un atome d'hydrogène du groupement protecteur G₁, lorsqu'il représente un radical trichloro-2,2,2 éthoxycarbonyle ou (trichlorométhyl-2 propoxy)-2 carbonyle est effectué par traitement par le zinc, éventuellement associé au cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone ou un ester aliphatique. tel que l'acétate d'éthyle, l'acétate d'isopropyle ou l'acétate de n.butyle en présence de zinc éventuellement associé à du cuivre, ou, lorsqu'il représente un radical alcoxyacétyle, par traitement en milieu alcalin au moyen d'ammoniac en milieu hydro-alcoolique à une température voisine de 20°C ou par traitement par un halogénure de zinc dans le méthanol à une température voisine de 20°C.

25. Procédé de préparation d'un produit selon l'une des revendications 1 à 5 **caractérisé en ce que** l'on estérifie un produit de formule générale : 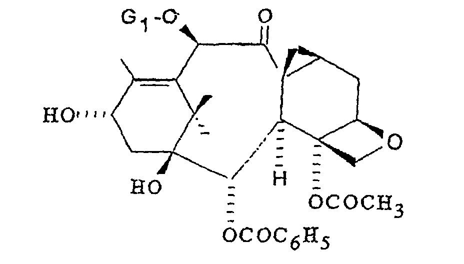 dans laquelle G₁ représente un atome d'hydrogène ou un radical acétyle ou un groupement protecteur de la fonction hydroxy, au moyen d'un acide de formule générale : 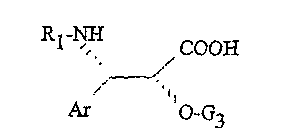 dans laquelle Ar et R₁ sont définis comme dans l'une des revendications 1 à 5 et G₃ représente un groupement protecteur de la fonction hydroxy, ou d'un dérivé activé de cet acide, pour obtenir un produit de formule générale : 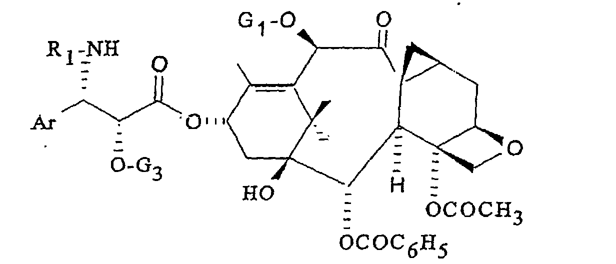 dans laquelle Ar, R₁, G₁ et G₃ sont définis comme précédemment, dont on remplace les groupements protecteurs G₃ et éventuellement G₁ par un atome d'hydrogène, et isole le produit obtenu.

26. Procédé selon la revendication 25 **caractérisé en ce que** l'estérification est effectuée au moyen de l'acide libre en opérant en présence d'un agent de condensation choisi parmi les carbodiimides et les carbonates réactifs et d'un agent d'activation choisi parmi les aminopyridines dans un solvant organique choisi parmi les éthers, les cétones, les esters, les nitriles, les hydrocarbures aliphatiques, les hydrocarbures aliphatiques halogénés et les hydrocarbures aromatiques à une température comprise entre -10 et 90°C.

27. Procédé selon la revendication 25 **caractérisé en ce que** l'estérification au moyen de l'anhydride est effectué en présence d'un agent d'activation choisi parmi les aminopyridines dans un solvant organique choisi parmi les éthers, les esters, les cétones, les nitriles, les hydrocarbures aliphatiques, les hydrocarbures aliphatiques halogénés et les hydrocarbures aromatiques à une température comprise entre 0 et 90°C.

28. Procédé selon la revendication 25 **caractérisé en ce que** l'estérification est effectuée au moyen d'un halogénure ou d'un anhydride avec un acide aliphatique ou aromatique, éventuellement préparé in situ, en opérant en présence d'une base choisie parmi les amines aliphatiques tertiaires dans un solvant organique choisi parmi les éthers, les esters, les cétones, les nitriles, les hydrocarbures aliphatiques, les hydrocarbures aliphatiques halogénés et les hydrocarbures aromatiques à une température comprise entre 0 et 80°C.

29. Procédé selon la revendication 25 **caractérisé en ce que** le remplacement des groupements protecteurs G₁ et G₃ par des atomes d'hydrogène est effectué par traitement par le zinc, éventuellement associé au cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone ou un ester aliphatique tel que l'acétate d'éthyle, l'acétate d'isopropyle ou l'acétate de n.butyle en présence de zinc éventuellement associé à du cuivre, lorsque G₁ et G₃ représentent un radical trichloro-2,2,2 éthoxycarbonyle ou (trichlorométhyl-2 propoxy)-2 carbonyle, ou par traitement en milieu acide tel que par exemple l'acide chlorhydrique en solution dans un alcool aliphatique contenant 1 à 3 atomes de carbone (méthanol, éthanol, propanol, isopropanol) ou l'acide fluorhydrique aqueux à une température comprise entre 0 et 40°C lorsque G₃ représente radical silylé ou un reste d'acétal, suivi du remplacement du groupement protecteur G₁ par traitement par le zinc, éventuellement associé au cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone ou un ester aliphatique tel que l'acétate d'éthyle, l'acétate d'isopropyle ou l'acétate de n.butyle en présence de zinc éventuellement associé à du cuivre ou, lorsque G₁ représente un radical alcoxyacétyle, par traitement en milieu alcalin au moyen d'ammoniac en milieu hydro-alcoolique à une température voisine de 20°C ou par traitement par un halogénure de zinc dans le méthanol à une température voisine de 20°C.

30. Procédé selon la revendication 25 **caractérisé en ce que**, lorsque G₃ représente un radical -CH₂-Ph, le remplacement du groupement par un atome d'hydrogène est effectué par hydrogénolyse, après avoir remplacé le groupement protecteur G₁ dans les conditions de la revendication 29.

31. Procédé de préparation d'un produit selon la revendication 1 **caractérisé en ce que** l'on fait réagir un halogénure de métal alcalin ou un azoture de métal alcalin ou un sel d'ammonium quaternaire ou un phosphate de métal alcalin sur un produit de formule générale : 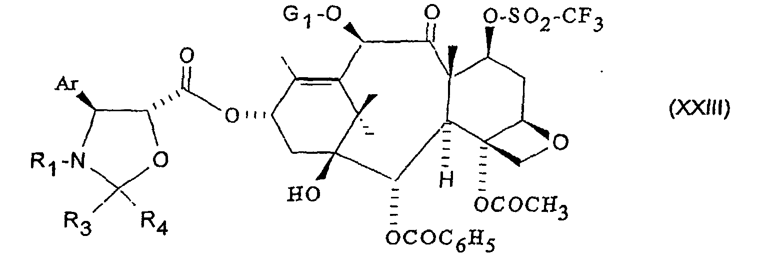 dans laquelle Ar et R₁ sont définis comme dans l'une des revendications 1, 2 ou 3 et G₁, R₃ et R₄ sont définis comme dans l'une des revendications 6 ou 14, pour obtenir un produit de formule générale : 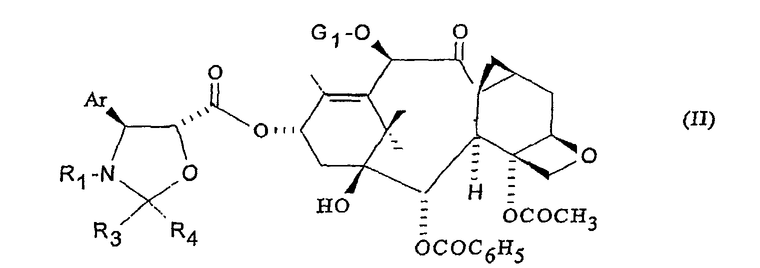 que l'on transforme en produit selon l'une des revendications 1, 2 ou 3 en opérant dans les conditions dela revendication 6

32. Taxoïde de formule générale : 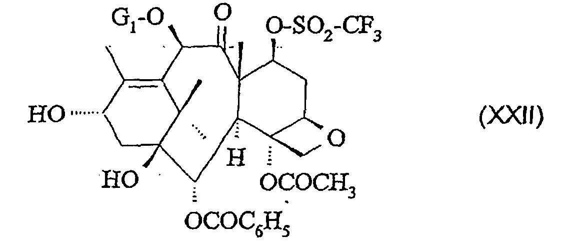 dans laquelle G₁ représente un atome d'hydrogène ou un radical acétyle, alcoxyacétyle ou un groupement protecteur de la fonction hydroxy.

33. Procédé de préparation du dérivé selon la revendication 32 de formule générale (XXII) **caractérisé en ce que** l'on fait réagir un dérivé de l'acide trifluoroacétique choisi parmi l'anhydride ou le phényltrifluorométhanesulfonimide sur la baccatine III ou la 10-déacétylbaccatine III.

34. Procédé selon la revendication 33 **caractérisé en ce que** la réaction est effectuée en présence d'une base organique.

35. Procédé selon la revendication 34 **caractérisé en ce que** la base organique est choisie parmi la triéthylamine et la pyridine.

36. Taxoïde de formule générale (XXIII): 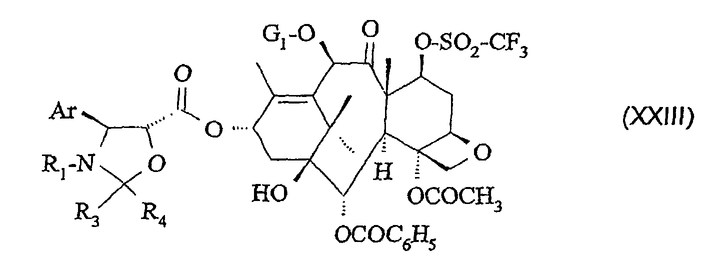 dans laquelle G₁ représente un atome d'hydrogène ou un radical acétyle, alcoxyacétyle ou un groupement protecteur de la fonction hydroxy, Ar et R₁ sont définis comme dans l'une des revendications 1 à 4 et R₃ et R₄ sont définis comme dans l'une des revendications 6 ou 14.

37. Procédé de préparation du produit de formule générale (XXIII) de la revendication 36 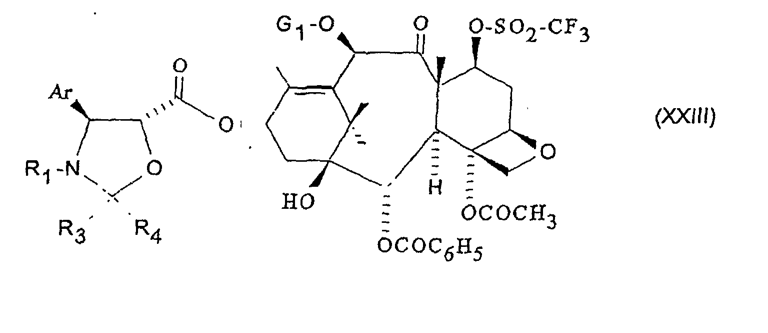 **caractérisé en ce que** l'on fait réagir un dérivé de l'acide fluorométhanesulfonique choisi parmi l'anhydride ou le N-phényltrifluorométhanesulfinide sur le produit de formule générale (XXTV) : 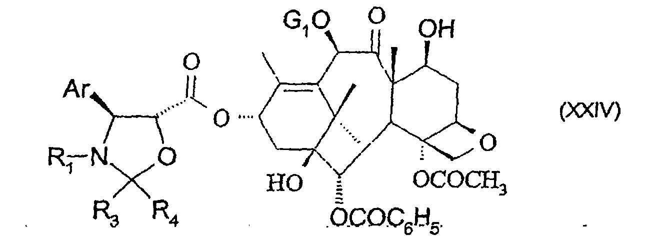 dans laquelle Ar, R1, R3 et R4 sont définis comme précédemment et GI représente un atome d'hydrogène ou un radical acétyle.

38. Procédé selon la revendication 37 **caractérisé en ce que** la réaction s'effectue dans un solvant organique inerte en présence d'une base organique.

39. Procédé selon la revendication 38 **caractérisé en ce que** la base est choisie parmi la txiéthylamine ou la pyridine.

40. Procédé selon les revendications 38 et 39 **caractérisé en ce que** la température est comprise entre -50 et +20°C.

41. Taxoïde de formule générale : 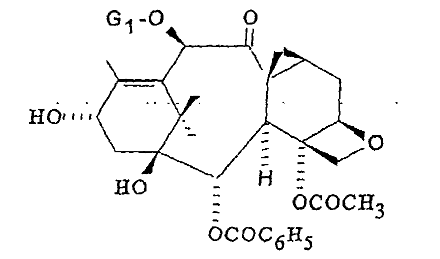 dans laquelle G₁ représente un atome d'hydrogène ou un radical acétyle, alcoxyacétyle ou alcoyle ou un groupement protecteur de la fonction hydroxy.

42. Taxoïdes de formule (II) 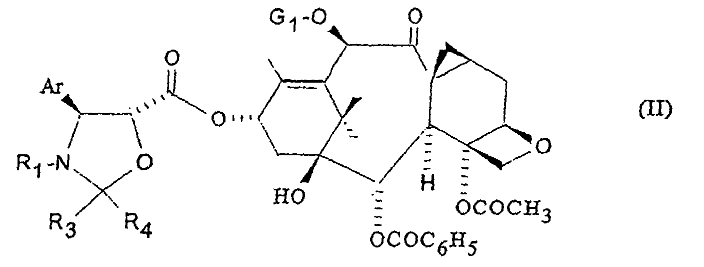 dans laquelle Ar, R₁, R₃, R₄ et G₁ sont définis comme à la revendication 6.

43. Procédé de préparation du produit de formule générale (II) 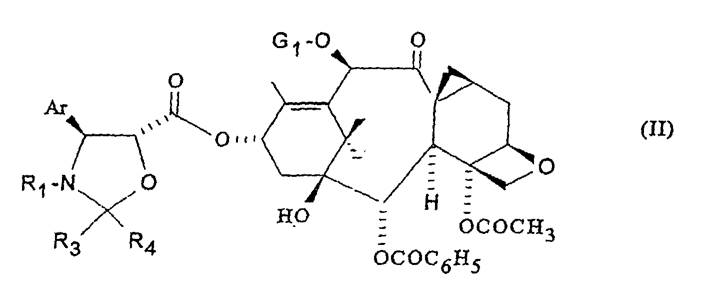 selon la revendication 42, dans laquelle Ar, R₁, R₃ et R₄ sont définis comme dans la revendication 42 et G₁ représente un atome d'hydrogène ou un radical acétyle,
**caractérisé en ce que** l'on procède à partir d'un produit de formule générale : 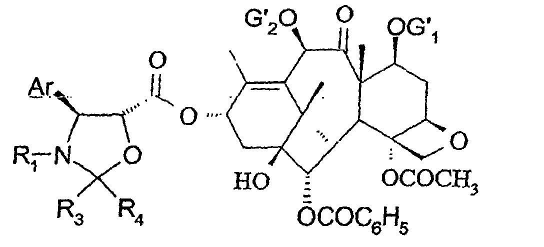 dans laquelle Ar, R₁, R₃ et R₄ sont définis comme dans la revendication 42, G'₁ représente un groupement protecteur de la fonction hydroxy et G'₂ représente un radical acétyle, alcoxyacétyle ou alcoyle ou un groupement protecteur de la fonction hydroxy,
- par remplacement des groupements protecteurs G'₁ et éventuellement G'₂ par des atomes d'hydrogène, afin d'obtenir le produit de formule générale : 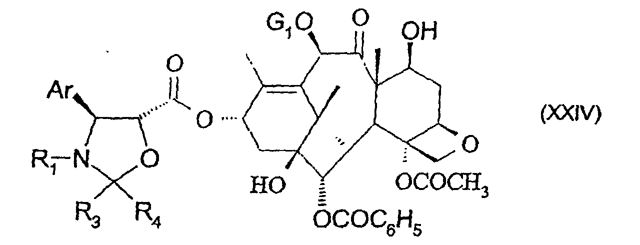 dans laquelle Ar, R₁, R₃, R₄ sont définis comme précédemment et G₁ représente un atome d'hydrogène ou un radical acétyle,
- suivi de l'action d'un dérivé de l'acide trifluorométhanesulfonique pour obtenir le produit de formule générale : 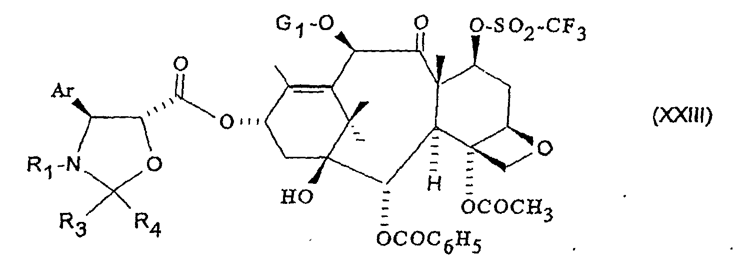
- et suivi de l'action d'un halogénure de métal alcalin, ou d'un azoture de métal alcalin, ou un sel d'ammonium quaternaire ou un phosphate de métal alcalin pour obtenir le produit de formule générale (II)

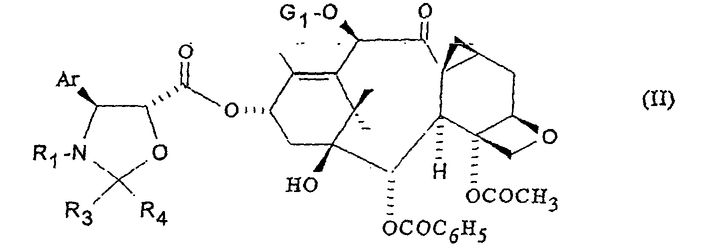

44. Procédé selon la revendication 43 **caractérisé en ce que** le remplacement des groupements protecteurs G'₁ et éventuellement G'₂ est réalisé par le zinc et l'acide acétique quand G'₁ et G'₂ sont tous les deux des groupes protecteurs.

45. Procédé selon la revendication 43 **caractérisé en ce que** le remplacement de G'₂ est effectué par l'acide chlorhydrique en solution éthanolique lorsque G'₂ est un groupe silylé.

46. Composition pharmaceutique **caractérisée en ce qu'**elle contient au moins un produit selon l'une des revendications 1 à 5 en association avec un ou plusieurs produits pharmaceutiquement acceptables qu'ils soient inertes ou physiologiquement actifs.

47. Utilisation des taxoïdes selon l'une des revendications 1 à 5 pour la préparation d'un médicament pour le traitement de laprolifération cellulaire anormale de cellules malignes ou non malignes de divers tissus et/ou organes, comprenant les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales et, pour le traitement des pathologies suivantes : le psoriasis, les tumeurs solides, les cancers de l'ovaire, du sein, du cerveau, de la prostate, du colon, de l'estomac, du rein ou des testicules, le sarcome de Kaposi, le cholangiocarcinome, le neuroblastome, la tumeur de Wilms, la maladie de Hodgkin, les mélanomes, les myélomes multiples, les leucémies lymphocytaires chroniques, les lymphomes granulocytaires aigus ou chroniques.

## Patentansprüche

1. Taxoide der allgemeinen Formel 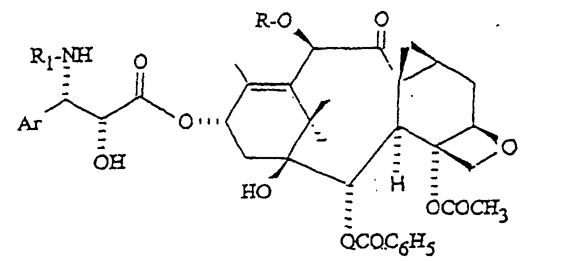 in der
R ein Wasserstoffatom oder einen Rest Acetyl, Alkoxyacetyl oder Alkyl darstellt,
R₁ einen Rest Benzoyl oder einen Rest R2-O-CO- bedeutet, worin R₂ ein Rest Alkyl, Alkenyl, Alkinyl, Cycloalkyl, Cycloalkenyl, Bicycloalkyl, Phenyl oder Heterocyclyl, gegebenenfalls substituiert, ist, und
Ar einen gegebenenfalls substituierten Rest Aryl darstellt.

2. Derivate nach Anspruch 1, worin
R ein Wasserstoffatom oder einen Rest Acetyl, Alkoxyacetyl oder Alkyl darstellt,
R₁ einen Rest Benzoyl oder einen Rest R₂-O-CO- bedeutet, worin R₂ darstellt:
- einen geraden oder verzweigten Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 10 Kohlenstoffatomen, wobei diese Reste gegebenenfalls substituiert sind durch einen oder mehrere, gleiche oder verschiedene Substituenten, ausgewählt unter den Halogenatomen und den Resten Hydroxy, Alkoxy mit 1 bis 4 Kohlenstoffatomen, Dialkylamino, bei dem jeder Teil Alkyl 1 bis 4 Kohlenstoffatome enthält, Piperidino, Morpholino, 1-Piperazinyl (gegebenenfalls substituiert in -4 durch einen Rest Alkyl mit 1 bis 4 Kohlenstoffatomen oder durch einen Rest Phenylalkyl, dessen Teil Alkyl 1 bis 4 Kohlenstoffatome enthält), Cycloalkyl mit 3 bis 6 Kohlenstoffatomen, Cycloalkenyl mit 4 bis 6 Kohlenstoffatomen, Phenyl, Cyano, Carboxy oder Alkoxycarbonyl, dessen Teil Alkyl 1 bis 4 Kohlenstoffatome enthält, oder
- einen Rest Phenyl, gegebenenfalls substituiert durch einen oder mehrere, gleiche oder verschiedene Reste, ausgewählt unter den Resten Alkyl mit 1 bis 4 Kohlenstoffatomen oder Alkoxy mit 1 bis 4 Kohlenstoffatomen, oder
- einen gesättigten oder ungesättigten Rest Stickstoff-Heterocyclyl mit 5 oder 6 Ringgliedern, gegebenenfalls substituiert durch einen oder mehrere Reste Alkyl mit 1 bis 4 Kohlenstoffatomen,
mit der Maßgabe, daß die Reste Cycloalkyl, Cycloalkenyl oder Bicycloalkyl gegebenenfalls substituiert sein können durch einen oder mehrere Reste Alkyl mit 1 bis 4 Kohlenstoffatomen, und Ar einen Rest Phenyl oder α- oder β-Naphthyl darstellt, gegebenenfalls substituiert durch ein oder mehrere Atome oder Reste, ausgewählt unter den Halogenatomen (Fluor, Chlor, Brom, Iod) und den Resten Alkyl, Alkenyl, Alkinyl, Aryl, Arylalkyl, Alkoxy, Alkylthio, Aryloxy, Arylthio, Hydroxy, Hydroxyalkyl, Mercapto, Formyl, Acyl, Acylamino, Aroylamino, Alkoxycarbonylamino, Amino, Alkylamino, Dialkylamino, Carboxy, Alkoxycarbonyl, Carbamoyl, Dialkylcarbamoyl, Cyano, Nitro und Trifluormethyl, mit der Maßgabe, daß die Reste Alkyl und die Teile Alkyl der anderen Reste 1 bis 4 Kohlenstoffatome enthalten, daß die Reste Alkenyl und Alkinyl 2 bis 8 Kohlenstoffatome enthalten und daß die Reste Aryl Reste Phenyl oder α- oder β-Naphthyl sind, oder
Ar einen aromatischen heterocyclischen Rest mit 5 Ringgliedern darstellt, der ein oder mehrere, gleiche oder verschiedene Atome enthält, ausgewählt unter den Atomen von Stickstoff, Sauerstoff oder Schwefel, gegebenenfalls substituiert durch einen oder mehrere; gleiche oder verschiedene Substituenten, ausgewählt unter den Halogenatomen (Fluor, Chlor, Brom, Iod) und den Resten Alkyl mit 1 bis 4 Kohlenstoffatomen, Aryl mit 6 bis 10 Kohlenstoffatomen, Alkoxy mit 1 bis 4 Kohlenstoffatomen, Aryloxy mit 6 bis 10 Kohlenstoffatomen, Amino, Alkylamino mit 1 bis 4 Kohlenstoffatomen, Dialkylamino, bei dem jeder Teil Alkyl 1 bis 4 Kohlenstoffatome enthält, Acylamino, dessen Teil Acyl 1 bis 4 Konlenstoffatome enthält, Alkoxycarbonylamino mit 1 bis 4 Kohlenstoffatomen, Acyl mit 1 bis 4 Kohlenstoffatomen, Arylcarbonyl, dessen Teil Aryl 6 bis 10 Kohlenstoffatome enthält, Cyano, Carboxy, Carbamoyl, Alkylcarbamoyl, dessen Teil Alkyl 1 bis 4 Kohlenstoffatome enthält, Dialkylcarbamoyl, bei dem jeder Teil Alkyl 1 bis 4 Kohlenstoffatome enthält, oder Alkoxycarbonyl, dessen Teil Alkoxy 1 bis 4 Kohlenstoffatome enthält.

3. Derivate nach Anspruch 1, worin R ein Wasserstoffatom oder einen Rest Acetyl, Alkoxyacetyl oder Alkyl darstellt, R₁ einen Rest Benzoyl oder einen Rest R₂-O-CO- bedeutet, worin R₂ ein Rest tert.-Butyl ist und Ar einen Rest Phenyl darstellt.

4. Derivate nach Anspruch 1, worin R einen Rest Acetyl darstellt, R₁ einen Rest R₂-O-CO- bedeutet, worin R₂ ein Rest tert.-Butyl ist und Ar einen Rest Phenyl darstellt.

5. Derivate nach Anspruch 1, worin R ein Wasserstoffatom darstellt, R₁ einen Rest R₂-O-CO- bedeutet, worin R₂ ein Rest tert.-Butyl ist und Ar einen Rest Phenyl darstellt.

6. Verfahren zur Herstellung eines Produktes nach einem der Ansprüche 1 bis 5, **dadurch gekennzeichnet, daß** man ein Produkt der allgemeinen Formel 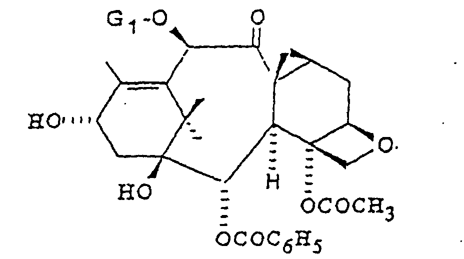 in der G₁ ein Wasserstoffatom oder einen Rest Acetyl, Alkoxyacetyl oder Alkyl oder eine Schutzgruppe für die Hydroxyfunktion darstellt, mit Hilfe einer Säure der allgemeinen Formel 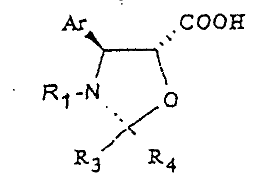 in der Ar und R₁ wie in einem der Ansprüche 1 bis 5 definiert sind, R₃ ein Wasserstoffatom oder einen Rest Alkoxy mit 1 bis 4 Kohlenstoffatomen oder einen gegebenenfalls substituierten Rest Aryl bedeutet und R₄ ein Wasserstoffatom ist, verestert, um ein Produkt der allgemeinen Formel (II) 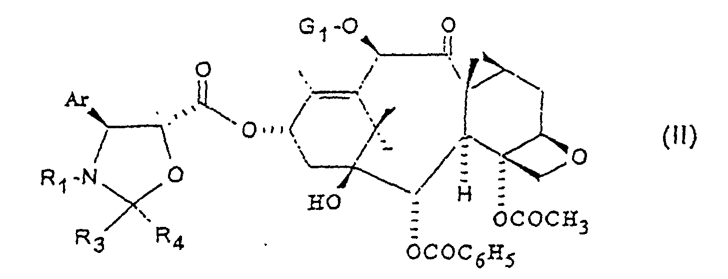 zu erhalten, in der Ar, R und R₁ wie in einem der Ansprüche 1 bis 5 definiert sind, R₃, R₄ und G₁ wie oben definiert sind, das man im sauren Medium behandelt, um ein Produkt der allgemeinen Formel 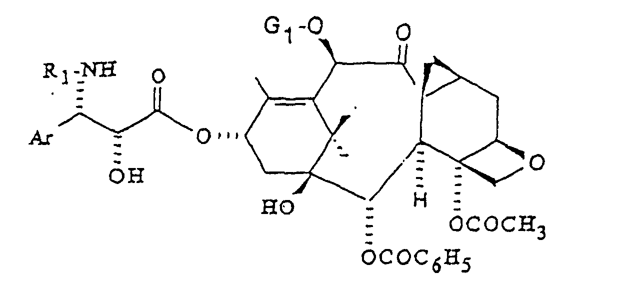 zu erhalten, in der Ar, R₁ und G₁ wie oben definiert sind, und man anschließend gegebenenfalls die Schutzgruppe G₁ durch ein Wasserstoffatom ersetzt und das erhaltene Produkt isoliert.

7. Verfahren nach Anspruch 6, **dadurch gekennzeichnet, daß** die Veresterung mit Hilfe von freier Säure durchgeführt wird, indem man in Anwesenheit eines Kondensationsmittels, ausgewählt unter den Carbodiimiden und den reaktiven Carbonaten, und eines Aktivierungsmittels, ausgewählt unter den Aminopyridinen, in einem organischen Lösungsmittel, ausgewählt unter den Ethern, den Ketonen, den Estern, den Nitrilen, den aliphatischen Kohlenwasserstoffen, den halogenierten aliphatischen Kohlenwasserstoffen und den aromatischen Kohlenwasserstoffen, sowie bei einer Temperatur zwischen -10 °C und 90 °C arbeitet.

8. Verfahren nach Anspruch 6, **dadurch gekennzeichnet, daß** die Veresterung mit Hilfe von Anhydrid in Anwesenheit eines Aktivierungsmittels, ausgewählt unter den Aminopyridinen, in einem organischen Lösungsmittel, ausgewählt unter den Ethern, den Estern, den Ketonen, den Nitrilen, den aliphatischen Kohlenwasserstoffen, den halogenierten aliphatischen Kohlenwasserstoffen und den aromatischen Kohlenwasserstoffen, sowie bei einer Temperatur zwischen 0 °C und 90 °C durchgeführt wird.

9. Verfahren nach Anspruch 6, **dadurch gekennzeichnet, daß** die Veresterung mit Hilfe eines Halogenides oder eines Anhydrides mit einer aliphatischen oder aromatischen Säure, gegebenenfalls in situ hergestellt, durchgeführt wird, indem man in Anwesenheit einer Base, ausgewählt unter den tertiären aliphatischen Aminen, in einem organischen Lösungsmittel, ausgewählt unter den Ethern, den Estern, den Ketonen, den Nitrilen, den aliphatischen Kohlenwasserstoffen, den halogenierten aliphatischen Kohlenwasserstoffen und den aromatischen Kohlenwasserstoffen, sowie bei einer Temperatur zwischen 0 °C und 80 °C arbeitet.

10. Verfahren nach Anspruch 6, **dadurch gekennzeichnet, daß** die saure Behandlung mit Hilfe einer Mineralsäure oder organischen Säure, in einem organischen Lösungsmittel sowie bei einer Temperatur zwischen -10 °C und 60 °C durchgeführt wird.

11. Verfahren nach Anspruch 10, **dadurch gekennzeichnet, daß** die Säure unter Chlorwasserstoffsäure, Schwefelsäure, Essigsäure, Methansulfonsäure, Trifluormethansulfonsäure und para-Toluolsulfonsäure, allein verwendet oder in Mischung, ausgewählt wird.

12. Verfahren nach Anspruch 10, **dadurch gekennzeichnet, daß** das Lösungsmittel unter den Alkoholen, den Ethern, den Estern, den halogenierten aliphatischen Kohlenwasserstoffen, den aromatischen Kohlenwasserstoffen und den Nitrilen ausgewählt wird.

13. Verfahren nach Anspruch 6, **dadurch gekennzeichnet, daß** der Austausch der Schutzgruppe G₁ durch ein Wasserstoffatom, wenn sie einen Rest 2,2,2-Trichlor-ethoxycarbonyl oder 2-(2-Trichlormethyl-propoxy)-carbonyl darstellt, durch Behandlung mit Zink, gegebenenfalls assoziiert mit Kupfer, in Anwesenheit von Essigsäure bei einer Temperatur zwischen 30 °C und 60 °C, oder mit Hilfe einer Mineralsäure oder organischen Säure wie Chlorwasserstoffsäure oder Essigsäure in Lösung eines aliphatischen Alkohols mit 1 bis 3 Kohlenstoffatomen oder eines aliphatischen Esters wie Ethylacetat, Isopropylacetat oder n-Butylacetat in Anwesenheit von Zink, gegebenenfalls assoziiert mit Kupfer, durchgeführt wird, und wenn sie einen Rest Alkoxyacetyl darstellt, durch Behandlung im alkalischen Medium mit Hilfe von Ammoniak im wäßrig-alkoholischen Medium bei einer Temperatur von etwa 20 °C oder durch Behandlung mit- einem Zinkhalogenid in Methanol bei einer Temperatur von etwa 20 °C erfolgt.

14. Verfahren zur Herstellung eines Produktes nach einem der Ansprüche 1 bis 5, **dadurch gekennzeichnet, daß** man ein Produkt der allgemeinen Formel 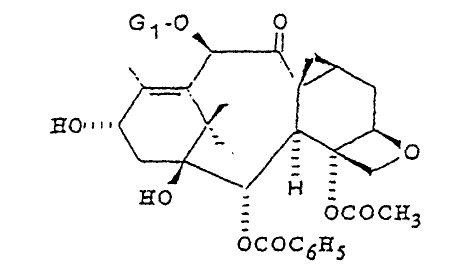 in der G₁ ein Wasserstoffatom oder einen Rest Acetyl, Alkoxyacetyl oder Alkyl oder eine Schutzgruppe für die Hydroxyfunktion darstellt, mit Hilfe einer Säure der allgemeinen Formel 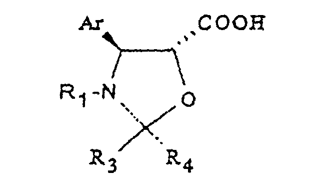 verestert, in der Ar und R₁ wie in einem der Ansprüche 1 bis 5 definiert sind und R₃ und R₄, gleich oder verschieden, einen Rest Alkyl mit 1 bis 4 Kohlenstoffatomen oder einen Rest Aralkyl, dessen Teil Alkyl 1 bis 4 Kohlenstoffatome enthält, oder einen Rest Aryl darstellen, oder auch R₃ einen Rest Trihalomethyl oder einen Rest Phenyl, substituiert durch einen Rest Trihalomethyl, bedeutet und R₄ ein Wasserstoffatom ist, oder auch R₃ und R₄ zusammen mit dem Kohlenstoffatom, an das sie gebunden sind, einen Ring mit 4 bis 7 Ringgliedern bilden, um nach Behandlung im sauren Medium ein Produkt der allgemeinen Formel 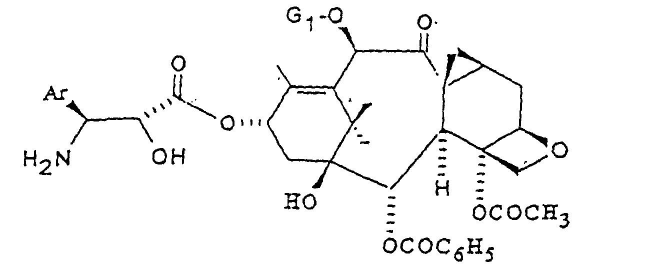 zu erhalten, in der Ar wie in einem der Ansprüche 1 bis 5 definiert ist und G₁ wie oben definiert ist, das mit Hilfe von Benzoylchlorid oder einem reaktiven Derivat der allgemeinen Formel
R₂-O-CO-X
in der R₂ wie in einem der Ansprüche 1 bis 5 definiert ist und X ein Halogenatom oder einen Rest -O-R₂ oder -O-CO-O-R₂ darstellt, acyliert wird, und man anschließend, wenn erforderlich, die Schutzgruppe G₁ durch ein Wasserstoffatom ersetzt und das erhaltene Produkt isoliert.

15. Verfahren nach Anspruch 14, **dadurch gekennzeichnet, daß** die Veresterung mit Hilfe von freier Säure durchgeführt wird, indem man in Anwesenheit eines Kondensationsmittels, ausgewählt unter den Carbodiimiden und den reaktiven Carbonaten, und eines Aktivierungsmittels, ausgewählt unter den Aminopyridinen, in einem organischen Lösungsmittel, ausgewählt unter den Ethern, den Ketonen, den Estern, den Nitrilen, den aliphatischen Kohlenwasserstoffen, den halogenierten aliphatischen Kohlenwasserstoffen und den aromatischen Kohlenwasserstoffen, sowie bei einer Temperatur zwischen -10 °C und 90 °C arbeitet.

16. Verfahren nach Anspruch 14, **dadurch gekennzeichnet, daß** die Veresterung mit Hilfe von Anhydrid in Anwesenheit eines Aktivierungsmittels, ausgewählt unter den Aminopyridinen, in einem organischen Lösungsmittel, ausgewählt unter den Ethern, den Estern, den Ketonen, den Nitrilen, den aliphatischen Kohlenwasserstoffen, den halogenierten aliphatischen Kohlenwasserstoffen und den aromatischen Kohlenwasserstoffen, sowie bei einer Temperatur zwischen 0 °C und 90 °C durchgeführt wird.

17. Verfahren nach Anspruch 14, **dadurch gekennzeichnet, daß** die Veresterung mit Hilfe eines Halogenides oder eines Anhydrides mit einer aliphatischen oder aromatischen Säure, gegebenenfalls in situ hergestellt, durchgeführt wird, indem man in Anwesenheit einer Base, ausgewählt unter den tertiären aliphatischen Aminen, in einem organischen Lösungsmittel, ausgewählt unter den Ethern, den Estern, den Ketonen, den Nitrilen, den aliphatischen Kohlenwasserstoffen; den halogenierten aliphatischen Kohlenwasserstoffen und den aromatischen Kohlenwasserstoffen, sowie bei einer Temperatur zwischen 0 °C und 80 °C arbeitet.

18. Verfahren nach Anspruch 14, **dadurch gekennzeichnet, daß** die saure Behandlung mit Hilfe einer Mineralsäure oder organischen Säure, in einem organischen Lösungsmittel sowie bei einer Temperatur zwischen 0 °C und 50 °C durchgeführt wird.

19. Verfahren nach Anspruch 18, **dadurch gekennzeichnet, daß** die Säure unter Chlorwasserstoffsäure, Schwefelsäure und Ameisensäure ausgewählt wird.

20. Verfahren nach Anspruch 18, **dadurch gekennzeichnet, daß** das Lösungsmittel unter den Alkoholen mit 1 bis 3 Kohlenstoffatomen ausgewählt wird.

21. Verfahren nach Anspruch 14, **dadurch gekennzeichnet, daß** die Acylierung in einem inerten organischen Lösungsmittel und in Anwesenheit einer Mineralbase oder organischen Base durchgeführt wird.

22. Verfahren nach Anspruch 21, **dadurch gekennzeichnet, daß** das inerte organische Lösungsmittel unter den Estern und den halogenierten aliphatischen Kohlenwasserstoffen ausgewählt wird.

23. Verfahren nach einem der Ansprüche 20, 21 oder 22, **dadurch gekennzeichnet, daß** man bei einer Temperatur, zwischen 0 °C und 50 °C arbeitet.

24. Verfahren nach Anspruch 14, **dadurch gekennzeichnet, daß** der Austausch der Schutzgruppe G₁ durch ein Wasserstoffatom, wenn sie einen Rest 2,2,2-Trichlor-ethoxycarbonyl oder 2-(2-Trichlormethyl-propoxy)-carbonyl darstellt, durch Behandlung mit Zink, gegebenenfalls assoziiert mit Kupfer, in Anwesenheit von Essigsäure bei einer Temperatur zwischen 30 °C und 60 °C, oder mit Hilfe einer Mineralsäure oder organischen Säure wie Chlorwasserstoffsäure oder Essigsäure in Lösung eines aliphatischen Alkohols mit 1 bis 3 Kohlenstoffatomen oder eines aliphatischen Esters wie Ethylacetat, Isopropylacetat oder n-Butylacetat in Anwesenheit von Zink, gegebenenfalls assoziiert mit Kupfer, durchgeführt wird, oder wenn sie einen Rest Alkoxyacetyl darstellt, durch Behandlung im alkalischen Medium mit Hilfe von Ammoniak im wäßrig-alkoholischen Medium bei einer Temperatur von etwa 20 °C oder durch Behandlung mit einem Zinkhalogenid in Methanol bei einer Temperatur von etwa 20 °C erfolgt.

25. Verfahren zur Herstellung eines Produktes nach einem der Ansprüche 1 bis 5, **dadurch gekennzeichnet, daß** man ein Produkt der allgemeinen Formel 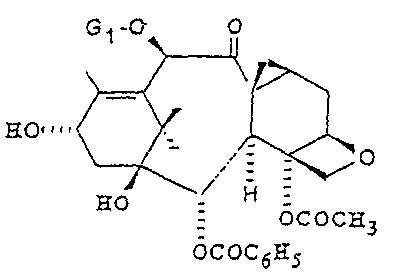 in der G₁ ein Wasserstoffatom oder einen Rest Acetyl oder eine Schutzgruppe für die Hydroxyfunktion darstellt, mit Hilfe einer Säure der allgemeinen Formel 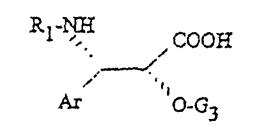 in der Ar und R₁ wie in einem der Ansprüche 1 bis 5 definiert sind und G₃ eine Schutzgruppe für die Hydroxyfunktion darstellt, oder einem aktivierten Derivat dieser Säure verestert, um ein Produkt der allgemeinen Formel 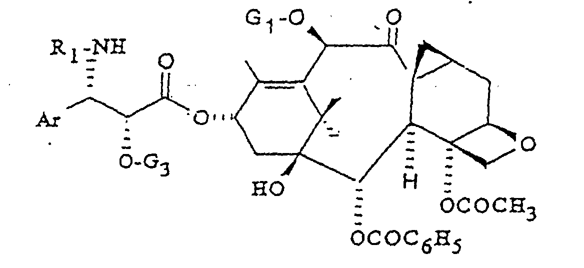 zu erhalten, in der Ar, R₁, G₁ und G₃ wie vorstehend definiert sind, bei dem man die Schutzgruppen G₃ und gegebenenfalls G₁ durch ein Wasserstoffatom ersetzt und das erhaltene Produkt isoliert.

26. Verfahren nach Anspruch 25, **dadurch gekennzeichnet, daß** die Veresterung mit Hilfe von freier Säure durchgeführt wird, indem man in Anwesenheit eines Kondensationsmittels, ausgewählt unter den Carbodiimiden und den reaktiven Carbonaten, und eines Aktivierungsmittels, ausgewählt unter den Aminopyridinen, in einem organischen Lösungsmittel, ausgewählt unter den Ethern, den Ketonen, den Estern, den Nitrilen, den aliphatischen Kohlenwasserstoffen, den halogenierten aliphatischen Kohlenwasserstoffen und den aromatischen Kohlenwasserstoffen, sowie bei einer Temperatur zwischen -10 °C und 90 °C arbeitet.

27. Verfahren nach Anspruch 25, **dadurch gekennzeichnet, daß** die Veresterung mit Hilfe von Anhydrid in Anwesenheit eines Aktivierungsmittels, ausgewählt unter den Aminopyridinen, in einem organischen Lösungsmittel, ausgewählt unter den Ethern, den Estern, den Ketonen, den Nitrilen, den aliphatischen Kohlenwasserstoffen, den halogenierten aliphatischen Kohlenwasserstoffen und den aromatischen Kohlenwasserstoffen, sowie bei einer Temperatur zwischen 0 °C und 90 °C durchgeführt wird.

28. Verfahren nach Anspruch 25, **dadurch gekennzeichnet, daß** die Veresterung mit Hilfe eines Halogenides oder eines Anhydrides mit einer aliphatischen oder aromatischen Säure, gegebenenfalls in situ hergestellt, durchgeführt wird, indem man in Anwesenheit einer Base, ausgewählt unter den tertiären aliphatischen Aminen, in einem organischen Lösungsmittel, ausgewählt unter den Ethern, den Estern, den Ketonen, den Nitrilen, den aliphatischen Kohlenwasserstoffen, den halogenierten aliphatischen Kohlenwasserstoffen und den aromatischen Kohlenwasserstoffen, sowie bei einer Temperatur zwischen 0 °C und 80 °C arbeitet.

29. Verfahren nach Anspruch 25, **dadurch gekennzeichnet, daß** der Austausch der Schutzgruppen G₁ und G₃ durch Wasserstoffatome, wenn G₁ und G₃ einen Rest 2,2,2-Trichlor-ethoxycarbonyl oder 2-(2-Trichlormethyl-propoxy)-carbonyl darstellen, durch Behandlung mit Zink, gegebenenfalls assoziiert mit Kupfer, in Anwesenheit von Essigsäure bei einer Temperatur zwischen 30 °C und 60 °C, oder mit Hilfe einer Mineralsäure oder organischen Säure wie Chlorwasserstoffsäure oder Essigsäure in Lösung eines aliphatischen Alkohols mit 1 bis 3 Kohlenstoffatomen oder eines aliphatischen Esters wie Ethylacetat, Isopropylacetat oder n-Butylacetat in Anwesenheit von Zink, gegebenenfalls assoziiert mit Kupfer, durchgeführt wird, oder wenn G₃ einen Rest Silyl oder einen Rest Acetal darstellt, durch Behandlung im sauren Medium wie beispielsweise Chlorwasserstoffsäure in Lösung eines aliphatischen Alkohols mit 1 bis 3 Kohlenstoffatomen (Methanol, Ethanol, Propanol, Isopropanol), oder wäßriger Fluorwasserstoffsäure bei einer Temperatur zwischen 0 °C und 40 °C, gefolgt von dem Austausch der Schutzgruppe G₁ durch Behandlung mit Zink, gegebenenfalls assoziiert mit Kupfer, in Anwesenheit von Essigsäure bei einer Temperatur zwischen 30 °C und 60 °C oder mit Hilfe einer Mineralsäure oder organischen Säure wie Chlorwasserstoffsäure oder Essigsäure in Lösung eines aliphatischen Alkohols mit 1 bis 3 Kohlenstoffatomen oder eines aliphatischen Esters wie Ethylacetat, Isopropylacetat oder n-Butylacetat in Anwesenheit von Zink, gegebenenfalls assoziiert mit Kupfer, vorgenommen wird, oder wenn G₁ einen Rest Alkoxyacetyl darstellt, durch Behandlung im alkalischen Medium mit Hilfe von Ammoniak im wäßrig-alkoholischen Medium bei einer Temperatur von etwa 20 °C oder durch Behandlung mit einem Zinkhalogenid in Methanol bei einer Temperatur von etwa 20 °C erfolgt.

30. Verfahren nach Anspruch 25, **dadurch gekennzeichnet, daß** in dem Fall, wo G₃ einen Rest -CH₂-Ph darstellt, der Austausch der Schutzgruppe durch ein Wasserstoffatom mittels Hydrogenolyse durchgeführt wird, nachdem der Austausch der Schutzgruppe G₁ unter den Bedingungen von Anspruch 29 erfolgte.

31. Verfahren zur Herstellung eines Produktes nach Anspruch 1, **dadurch gekennzeichnet, daß** man ein Alkalimetallhalogenid oder ein Alkalimetallazid oder ein quaternäres Ammoniumsalz oder ein Alkalimetallphosphat mit einem Produkt der allgemeinen Formel (XXIII) 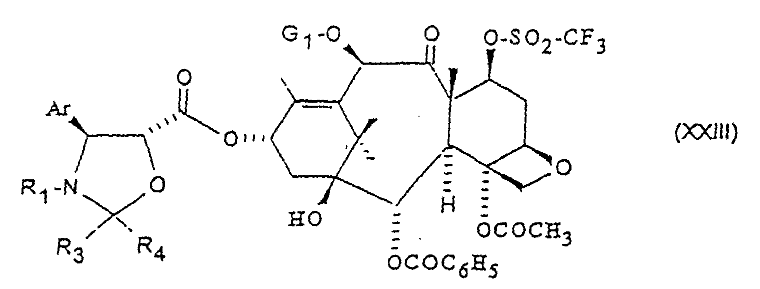 in der Ar und R₁ wie in einem der Ansprüche 1, 2 oder 3 definiert sind und G₁, R₃ und R₄ wie in einem der Ansprüche 6 oder 14 definiert sind, zur Reaktion bringt, um ein Produkt der allgemeinen Formel (II) 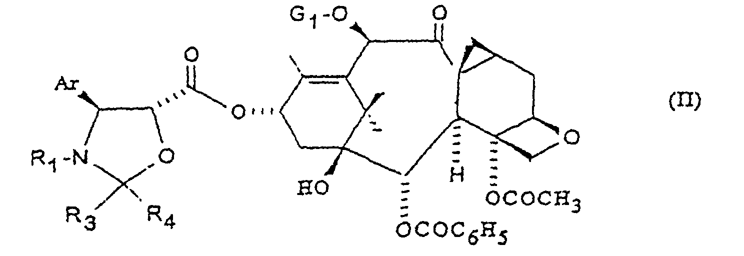 zu erhalten, das man in ein Produkt nach einem der Ansprüche 1, 2 oder 3 umwandelt, indem man unter den Bedingungen von Anspruch 6 arbeitet.

32. Taxoid der allgemeinen Formel (XXII) 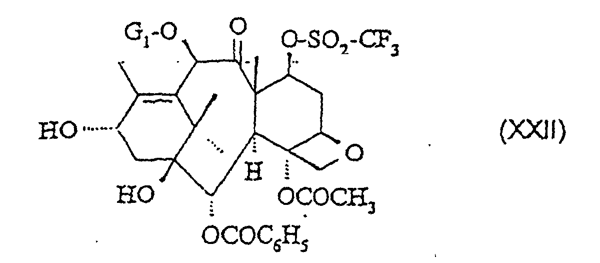 in der G₁ ein Wasserstoffatom oder einen Rest Acetyl, Alkoxyacetyl oder eine Schutzgruppe für die Hydroxyfunktion darstellt.

33. Verfahren zur Herstellung des Derivates der allgemeinen Formel (XXII) nach Anspruch 32, **dadurch gekennzeichnet, daß** man ein Derivat der Trifluoressigsäure, ausgewählt unter dem Anhydrid oder dem Phenyltrifluormethansulfonimid, mit Baccatin III oder 10-Desacetylbaccatin III zur Reaktion bringt.

34. Verfahren nach Anspruch 33, **dadurch gekennzeichnet, daß** die Reaktion in Anwesenheit einer organischen Base durchgeführt wird.

35. Verfahren nach Anspruch 34, **dadurch gekennzeichnet, daß** die organische Base unter Triethylamin und Pyridin ausgewählt wird.

36. Taxoid der allgemeinen Formel (XXIII) 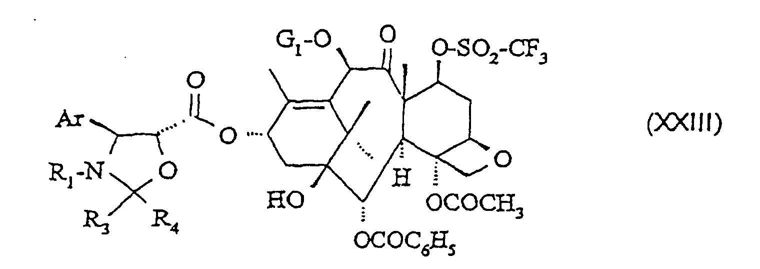 in der G₁ ein Wasserstoffatom oder einen Rest Acetyl, Alkoxyacetyl oder eine Schutzgruppe für die Hydroxyfunktion darstellt, Ar und R₁ wie in einem der Ansprüche 1 bis 4 definiert sind und R₃ und R₄ wie in einem der Ansprüche 6 oder 14 definiert sind.

37. Verfahren zur Herstellung des Produktes der allgemeinen Formel (XXIII) von Anspruch 36 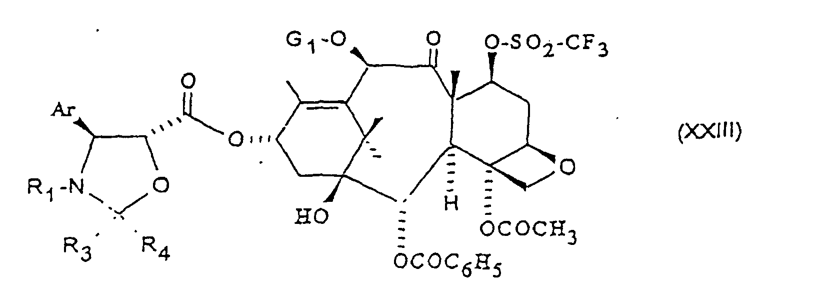 **dadurch gekennzeichnet, daß** man ein Derivat von Fluormethansulfonsäure, ausgewählt unter dem Anhydrid oder dem N-Phenyltrifluormethansulfimid, mit dem Produkt der allgemeinen Formel (XXIV) 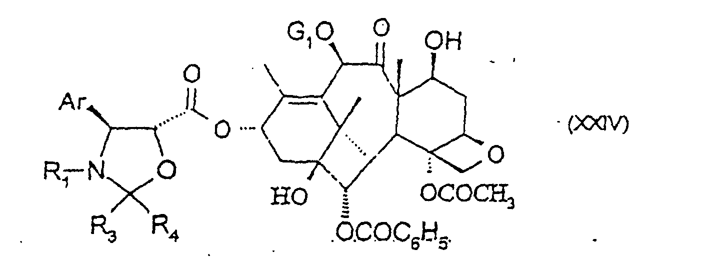 in der Ar, R₁, R₃ und R₄ wie vorstehend definiert sind und G₁ ein Wasserstoffatom oder einen Rest Acetyl darstellt, zur Reaktion bringt.

38. Verfahren nach Anspruch 37, **dadurch gekennzeichnet, daß** die Reaktion in einem inerten organischen Lösungsmittel und in Anwesenheit einer organischen Base durchgeführt wird.

39. Verfahren nach Anspruch 38, **dadurch gekennzeichnet, daß** die organische Base unter Triethylamin oder Pyridin ausgewählt wird.

40. Verfahren nach Anspruch 38 und 39, **dadurch gekennzeichnet, daß** die Temperatur zwischen -50 °C und +20 °C beträgt.

41. Taxoid der allgemeinen Formel 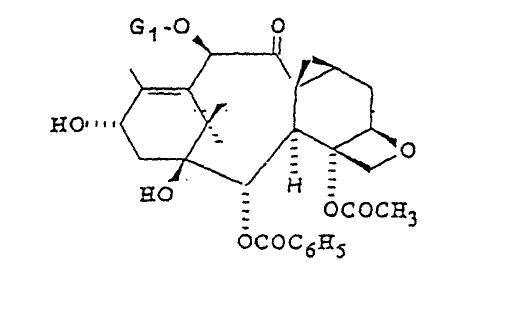 in der G₁ ein Wasserstoffatom oder einen Rest Acetyl, Alkoxyacetyl oder Alkyl oder eine Schutzgruppe für die Hydroxyfunktion darstellt.

42. Taxoide der Formel (II) 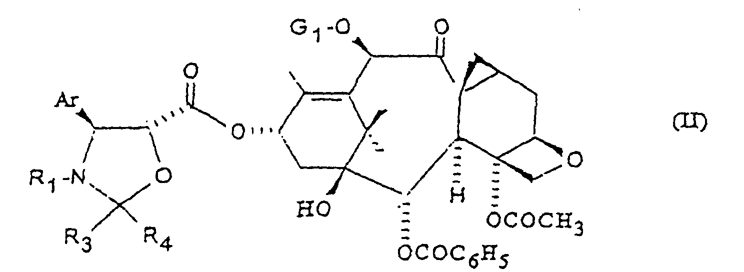 in der Ar, R₁, R₃, R₄ und G₁ wie in Anspruch 6 definiert sind.

43. Verfahren zur Herstellung des Produktes der allgemeinen Formel (II) nach Anspruch 42 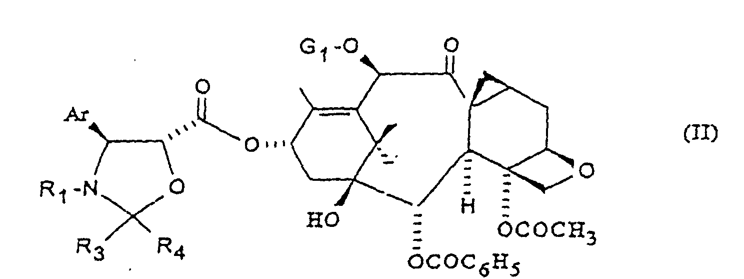 in der Ar, R₁, R₃ und R₄ wie in Anspruch 42 definiert sind und G₁ ein Wasserstoffatom oder einen Rest Acetyl darstellt, **dadurch gekennzeichnet, daß** man ausgehend von einem Produkt der allgemeinen Formel 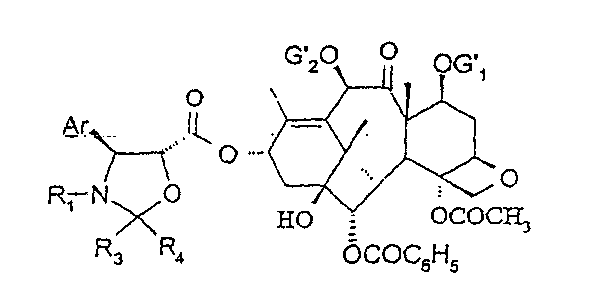 in der Ar, R₁, R₃ und R₄ wie in Anspruch 42 definiert sind, G'₁ eine Schutzgruppe für die Hydroxyfunktion ist und G'₂ einen Rest Acetyl, Alkoxyacetyl oder Alkyl oder eine Schutzgruppe für die Hydroxyfunktion darstellt,
- den Austausch der Schutzgruppen G'₁ und gegebenenfalls G'₂ durch Wasserstoffatome vornimmt, um das Produkt der allgemeinen Formel (XXIV) 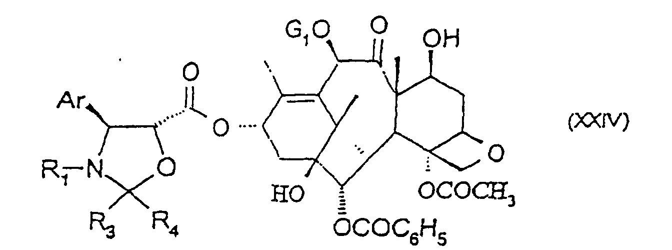 zu erhalten, in der Ar, R₁, R₃ und R₄ wie vorstehend definiert sind und G₁ ein Wasserstoffatom oder einen Rest Acetyl darstellt,
- danach die Einwirkung eines Derivates von Trifluormethansulfonsäure durchführt, um das Produkt der allgemeinen Formel (XXIII) 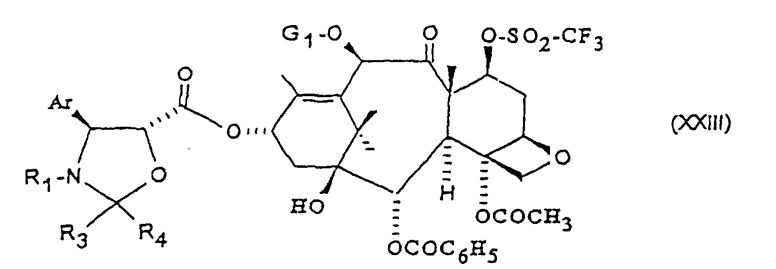 zu erhalten, und
- anschließend die Einwirkung eines Alkalimetallhalogenides oder eines Alkalimetallazides oder eines quaternären Ammoniumsalzes oder eines Alkalimetallphosphates vornimmt, um das Produkt der allgemeinen Formel (II) 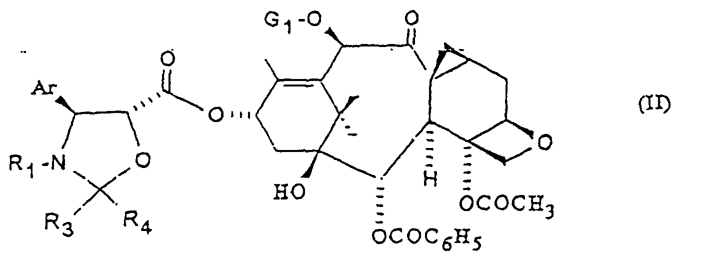 zu erhalten.

44. Verfahren nach Anspruch 43, **dadurch gekennzeichnet, daß** der Austausch der Schutzgruppen G'₁ und gegebenenfalls G'₂ durch Zink und Essigsäure realisiert wird, wenn G'₁ und G'₂ beides Schutzgruppen sind.

45. Verfahren nach Anspruch 43, **dadurch gekennzeichnet, daß** der Austausch von G'₂ mittels Chlorwasserstoffsäure in ethanolischer Lösung durchgeführt wird, wenn G'₂ eine Gruppe Silyl ist.

46. Pharmazeutische Zusammensetzung, **dadurch gekennzeichnet, daß** sie mindestens ein Produkt nach einem der Ansprüche 1 bis 5 in Assoziation mit einem oder mehreren pharmazeutisch akzeptablen Produkten enthält, die inert oder physiologisch aktiv sind.

47. Verwendung der Taxoide nach einem der Ansprüche 1 bis 5 zur Herstellung eines Arzneimittels für die Behandlung der anormalen zellularen Proliferation von malignen oder nicht malignen Zellen verschiedener Gewebe und/oder Organe, umfassend die muskulären Gewebe, Knochengewebe oder Bindegewebe, die Haut, das Gehirn, die Lunge, die Sexualorgane, die lymphatischen Systeme oder die Nierensysteme, die Brust- oder Blutzellen, die Leber, den Verdauungsapparat, die Bauchspeicheldrüse und die Schilddrüse oder adrenale Drüse, sowie für die Behandlung der folgenden Erkrankungen: Psoriasis, feste Tumore, Krebszustände der Eierstöcke, der Brust, des Gehirns, der Prostata, des Dickdarms, des Magens, der Nieren oder der Hoden, des Sarcoms nach Kaposi, des Cholangiokarzinoms, des Neuroblastoms, des Tumors nach Wilms, der Hodgin-Erkrankung, von Melanomen, von multiplen Myelomen, von chronischer lymphozitärer Leukämie, von akuten oder chronischen granulozytären Lymphomen.

## Claims

1. Taxoids of general formula: 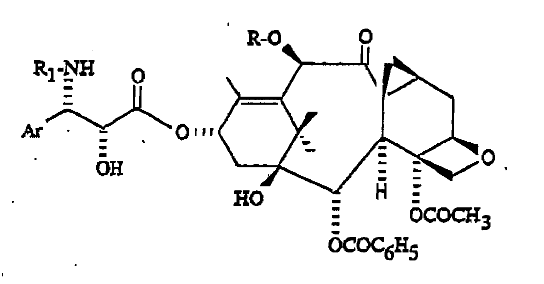 in which
R represents a hydrogen atom or an acetyl, alkoxyacetyl or alkyl radical,
R₁ represents a benzoyl radical or a radical R₂-O-CO- in which R₂ represents an optionally substituted alkyl, alkenyl, alkynyl, cycloalkyl, cycloalkenyl, bicycloalkyl, phenyl or heterocyclyl radical, and
Ar represents an optionally substituted aryl radical.

2. Derivatives according to Claim 1 for which:
R represents a hydrogen atom or an acetyl, alkoxyacetyl or alkyl radical,
R₁ represents a benzoyl radical or a radical R₂-O-CO in which R₂ represents :
- a straight or branched alkyl radical containing 1 to 8 carbon atoms, an alkenyl radical containing 2 to 8 carbon atoms, an alkylnyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals being optionally substituted by one or more substituents, which are identical or different, chosen from halogen atoms and hydroxy radicals, alkoxy radicals containing 1 to 4 carbon atoms, dialkylamino radicals in which each alkyl portion contains 1 to 4 carbon atoms, piperidino radicals, morpholino radicals, 1-piperazinyl radicals (optionally substituted at position 4 by an alkyl radical containing 1 to 4 carbon atoms or by a phenylalkyl radical whose alkyl portion contains 1 to 4 carbon atoms), cycloalkyl radicals containing 3 to 6 carbon atoms, cycloalkenyl radicals containing 4 to 6 carbon atoms, phenyl radicals, cyano radicals, carboxy radicals or alkoxycarbonyl radicals whose alkyl portion contains 1 to 4 carbon atoms,
- or a phenyl radical optionally substituted by one or more radicals, which are identical or different, chosen from alkyl radicals containing 1 to 4 carbon atoms or alkoxy radicals containing 1 to 4 carbon atoms,
- or a saturated or unsaturated 5- or 6-membered nitrogen-containing heterocyclyl radical optionally substituted by one or more alkyl radicals containing 1 to 4 carbon atoms,
it being understood that the cycloalkyl, cycloalkenyl or bicycloalkyl radicals may be optionally substituted by one or more alkyl radicals containing 1 to 4 carbon atoms, and
Ar represents a phenyl or α- or β-naphthyl radical optionally substituted by one or more atoms or radicals, chosen from halogen, atoms (fluorine, chlorine, bromine, or iodine) and alkyl, alkenyl, alkynyl, aryl, arylalkyl, alkoxy, alkylthio, aryloxy, arylthio, hydroxy, hydroxyalkyl, mercapto, formyl, acyl, acylamino, aroylamino, alkoxycarbonylamino, amino, alkylamino, dialkylamino, carboxy, alkoxycarbonyl, carbamoyl, dialkylcarbamoyl, cyano, nitro and trifluoromethyl radicals, it being understood that the alkyl radicals and the alkyl portions of the other radicals contain 1 to 4 carbon atoms, that the alkenyl and alkynyl radicals contain 2 to 8 carbon atoms and that the aryl radicals are phenyl or α- or β-naphthyl radicals or alternatively Ar represents a 5-membered aromatic heterocyclic radical containing one or more atoms, which are identical or different, chosen from nitrogen, oxygen or sulphur atoms, optionally substituted by one or more substituents, which are identical or different, chosen from halogen atoms (fluorine, chlorine, bromine or iodine) and alkyl radicals containing 1 to 4 carbon atoms, aryl radicals containing 6 to 10 carbon atoms, alkoxy radicals containing 1 to 4 carbon atoms, aryloxy radicals containing 6 to 10 carbon atoms, amino radicals, alkylamino radicals containing 1 to 4 carbon atoms, dialkylamino radicals in which each alkyl portion contains 1 to 4 carbon atoms, acylamino radicals in which the acyl portion contains 1 to 4 carbon atoms, alkoxycarbonylamino radicals containing 1 to 4 carbon atoms, acyl radicals containing 1 to 4 carbon atoms, arylcarbonyl radicals in which the aryl portion contains 6 to 10 carbon atoms, cyano radicals, carboxy radicals, carbamoyl radicals, alkylcarbamoyl radicals in which the alkyl portion contains 1 to 4 carbon atoms, dialkylcarbamoyl radicals in which each alkyl portion contains 1 to 4 carbon atoms or alkoxycarbonyl radicals in which the alkoxy portion contains 1 to 4 carbon atoms.

3. Derivatives according to Claim 1, for which R represents a hydrogen atom or an acetyl, alkoxyacetyl or alkyl radical, R₁ represents a benzoyl radical or a radical R₂-O-CO- in which R₂ represents a t-butyl radical and Ar represents a phenyl radical.

4. Derivative according to Claim 1, for which R represents an acetyl radical, R₁ represents a radical R₂-O-CO- in which R₂ represents a t-butyl radical and Ar represents a phenyl radical.

5. Derivative according to Claim 1, for which R represents a hydrogen atom, R₁ represents a radical R₂-O-CO- in which R₂ represents a t-butyl radical and Ar represents a phenyl radical.

6. Process for the preparation of a product according to one of Claims 1 to 5, **characterized in that** a product of general formula: 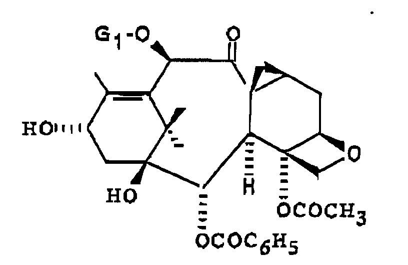 in which G₁ represents a hydrogen atom or an acetyl, alkoxyacetyl or alkyl radical or a hydroxy-protecting group, is esterified by means of an acid of general formula: 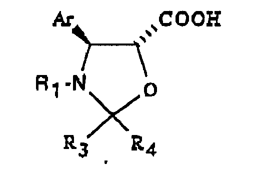 in which Ar and R₁ are defined as in one of Claims 1 to 5, R₃ represents a hydrogen atom or an alkoxy radical containing 1 to 4 carbon atoms or an optionally substituted aryl radical and R₄ represents a hydrogen atom, to give a product of general formula: 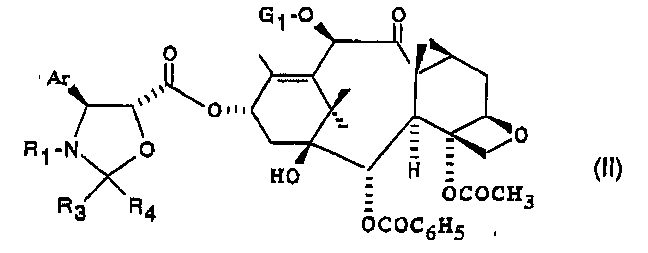 in which Ar, R and R₁ are defined as in one of Claims 1 to 5, R₃, R₄ and G₁ are defined as above, which is treated in acidic medium to give a product of general formula: 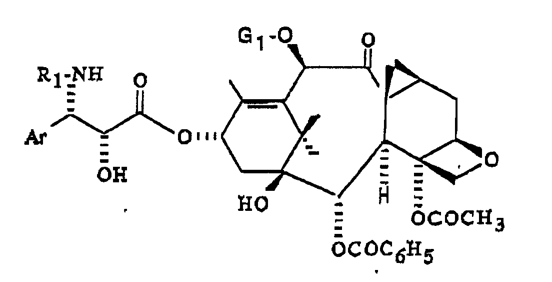 in which Ar, R₁ and G₁ are defined as above, and then the protecting group G₁ is optionally replaced by a hydrogen atom and the product obtained is isolated.

7. Process according to Claim 6, **characterized in that** the esterification is carried out by means of the free acid, the procedure being carried out in the presence of a condensing agent chosen from carbodiimides and reactive carbonates and an activating agent chosen from aminopyridines in an organic solvent chosen from ethers, ketones, esters, nitriles, aliphatic hydrocarbons, halogenated aliphatic hydrocarbons and aromatic hydrocarbons at a temperature of between -10 and 90°C.

8. Process according to Claim 6, **characterized in that** the esterification by means of the anhydride is carried out in the presence of an activating agent chosen from aminopyridines in an organic solvent chosen from ethers, esters, ketones, nitriles, aliphatic hydrocarbons, halogenated aliphatic hydrocarbons and aromatic hydrocarbons at a temperature of between 0 and 90°C.

9. Process according to Claim 6, **characterized in that** the esterification is carried out by means of a halide or an anhydride with an aliphatic or aromatic acid, optionally prepared in situ, the procedure being carried out in the presence of a base chosen from tertiary aliphatic amines in an organic solvent chosen from ethers, esters, ketones, nitriles, aliphatic hydrocarbons, halogenated aliphatic hydrocarbons and aromatic hydrocarbons at a temperature of between 0 and 80°C.

10. Process according to Claim 6, **characterized in that** the acid treatment is carried out by means of an inorganic or organic acid in an organic solvent at a temperature of between -10 and 60°C.

11. Process according to Claim 10, **characterized in that** the acid is chosen from hydrochloric, sulphuric, acetic, methanesulphonic, trifluoromethanesulphonic and p-toluenesulphonic acids, used alone or in the form of a mixture.

12. Process according to Claim 10, **characterized in that** the solvent is chosen from alcohols, ethers, esters, halogenated aliphatic hydrocarbons, aromatic hydrocarbons and nitriles.

13. Process according to Claim 6, **characterized in that** the replacement by a hydrogen atom of the protecting group G₁, when it represents a 2,2,2-trichloroethoxycarbonyl or 2-(2-trichloromethylpropoxy)carbonyl radical, is carried out by treatment using zinc, optionally combined with copper, in the presence of acetic acid at a temperature of between 30 and 60°C or by means of an inorganic or organic acid such as hydrochloric acid or acetic acid in solution in an aliphatic alcohol containing 1 to 3 carbon atoms or an aliphatic ester such as ethyl acetate, isopropyl acetate or n-butyl acetate in the presence of zinc optionally combined with copper, and, when it represents an alkoxyacetyl radical, by treatment in alkaline medium by means of ammonia in aqueous-alcoholic medium at a temperature close to 20°C or by treatment using a zinc halide in methanol at a temperature close to 20°C.

14. Process for the preparation of a product according to one of Claims 1 to 5, **characterized in that** a product of general formula: 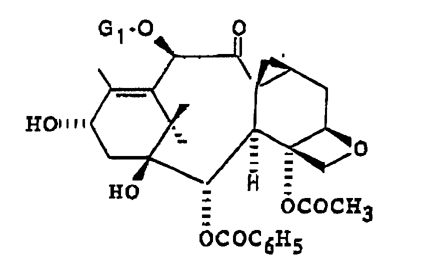 in which G₁ represents a hydrogen atom or an acetyl, alkokyacetyl or alkyl radical or a hydroxy-protecting group, is esterified by means of an acid of general formula: 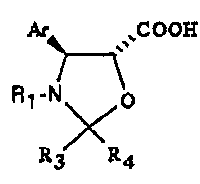 in which Ar and R₁ are defined as in one of Claims 1 to 5 and R₃ and R₄, which are identical or different, represent an alkyl radical containing 1 to 4 carbon atoms or an aralkyl radical whose alkyl portion contains 1 to 4 carbon atoms or an aryl radical, or alternatively R₃ represents a trihalomethyl radical or a phenyl radical substituted by a trihalomethyl radical and R₄ represents a hydrogen atom, or alternatively R₃ and R₄ form, together with the carbon atom to which they are attached, a 4- to 7-membered ring, to give, after treatment in acidic medium, a product of general formula: 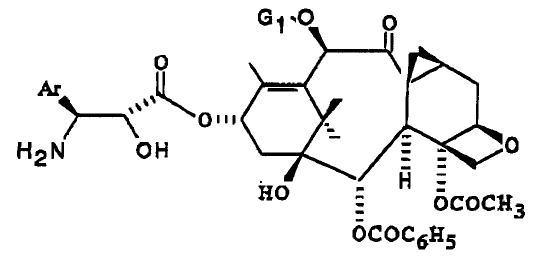 in which Ar is defined as in one of Claims 1 to 5 and G₁ is defined as above, which is acylated by means of benzoyl chloride or a reactive derivative of general formula:
R₂-O-CO-X
in which R₂ is defined as in one of Claims 1 to 5 and X represents a halogen atom or a residue -O-R₂ or -O-CO-O-R₂, and then the protecting group G₁ is replaced, if necessary, by a hydrogen atom, and the product obtained is isolated.

15. Process according to Claim 14, **characterized in that** the esterification is carried out by means of the free acid, the procedure being carried out in the presence of a condensing agent chosen from carbodiimides and reactive carbonates and an activating agent chosen from aminopyridines in an organic solvent chosen from ethers, ketones, esters, nitriles, aliphatic hydrocarbons, halogenated aliphatic hydrocarbons and aromatic hydrocarbons at a temperature of between -10 and 90°C.

16. Process according to Claim 14, **characterized in that** the esterification by means of the anhydride is carried out in the presence of an activating agent chosen from aminopyridines in an organic solvent chosen from ethers, esters, ketones, nitriles, aliphatic hydrocarbons, halogenated aliphatic hydrocarbons and aromatic hydrocarbons at a temperature of between 0 and 90°C.

17. Process according to Claim 14, **characterized in that** the esterification is carried out by means of a halide or an anhydride with an aliphatic or aromatic acid, optionally prepared in situ, the procedure being carried out in the presence of a base chosen from tertiary aliphatic amines in an organic solvent chosen from ethers, esters, ketones, nitriles, aliphatic hydrocarbons, halogenated aliphatic hydrocarbons and aromatic hydrocarbons at a temperature of between 0 and 80°C.

18. Process according to Claim 14, **characterized in that** the acid treatment is carried out by means of an inorganic or organic acid in an organic solvent at a temperature of between 0 and 50°C.

19. Process according to Claim 18, **characterized in that** the acid is chosen from hydrochloric, sulphuric and formic acids.

20. Process according to Claim 18, **characterized in that** the solvent is chosen from alcohols containing 1 to 3 carbon atoms.

21. Process according to Claim 14, **characterized in that** the acylation is carried out in an inert organic solvent in the presence of an inorganic or organic base.

22. Process according to Claim 21, **characterized in that** the inert organic solvent is chosen from esters and halogenated aliphatic hydrocarbons.

23. Process according to one of Claims 20, 21 or 22, **characterized in that** the procedure is carried out at a temperature of between 0 and 50°C.

24. Process according to Claim 14, **characterized in that** the replacement by a hydrogen atom of the protecting group G₁, when it represents a 2,2,2-trichloroethoxycarbonyl or 2-(2-trichloromethylpropoxy)carbonyl radical, is carried out by treatment using zinc, optionally combined with copper, in the presence of acetic acid at a temperature of between 30 and 60°C or by means of an inorganic or organic acid such as hydrochloric acid or acetic acid in solution in an aliphatic alcohol containing 1 to 3 carbon atoms or an aliphatic ester such as ethyl acetate, isopropyl acetate or n-butyl acetate in the presence of zinc optionally combined with copper, or, when it represents an alkoxy acetyl radical, by treatment in alkaline medium by means of ammonia in aqueous-alcoholic medium at a temperature close to 20°C or by treatment using a zinc halide in methanol at a temperature close to 20°C.

25. Process the preparation of product according to one of Claims 1 to 5, **characterized in that** a product of general formula: 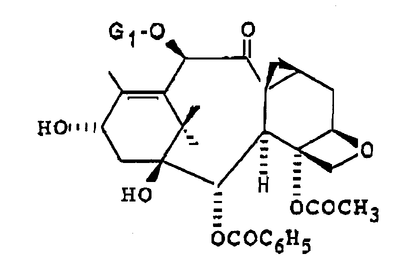 in which G₁ represents a hydrogen atom or an acetyl radical or a hydroxy-protecting group, is esterified by means of an acid of general formula: 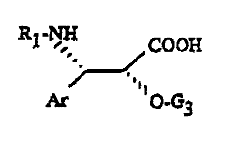 in which Ar and R₁ are defined as in one of Claims 1 to 5 and G₃ represents a hydroxy-protecting group, or of an activated derivative of this acid, to give a product of general formula: 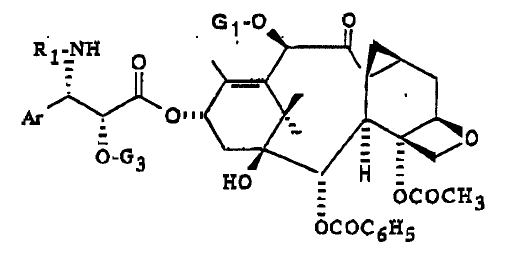 in which Ar, R₁, G₁ and G₃ are defined as above, whose protecting groups G₃ and optionally G₁ are replaced by a hydrogen atom, and the product obtained is isolated.

26. Process according to Claim 25, **characterized in that** the esterification is carried out by means of the free acid, the procedure being carried out in the presence of a condensing agent chosen from carbodiimides and reactive carbonates and an activating agent chosen from aminopyridines in an organic solvent chosen from ethers, ketones, esters, nitriles, aliphatic hydrocarbons, halogenated aliphatic hydrocarbons and aromatic hydrocarbons at a temperature of between -10 and 90°C.

27. Process according to Claim 25, **characterized in that** the esterification by means of the anhydride is carried out in the presence of an activating agent chosen from aminopyridines in an organic solvent chosen from ethers, esters, ketones, nitriles, aliphatic hydrocarbons, halogenated aliphatic hydrocarbons and aromatic hydrocarbons at a temperature of between 0 and 90°C.

28. Process according to Claim 25, **characterized in that** the esterification is carried out by means of a halide or an anhydride with an aliphatic or aromatic acid, optionally prepared in situ, the procedure being carried out in the presence of a base chosen from tertiary aliphatic amines in an organic solvent chosen from ethers, esters, ketones, nitriles, aliphatic hydrocarbons, halogenated aliphatic hydrocarbons and aromatic hydrocarbons at a temperature of between 0 and 80°C.

29. Process according to Claim 25, **characterized in that** the replacement of the protecting groups G₁ and G₃ by hydrogen atoms is carried out by treatment with zinc, optionally combined with copper, in the presence of acetic acid at a temperature of between 30 and 60°C or by means of an inorganic or organic acid such as hydrochloric acid or acetic acid in solution in an aliphatic alcohol containing 1 to 3 carbon atoms or an aliphatic ester such as ethyl acetate, isopropyl acetate or n-butyl acetate in the presence of zinc optionally combined with copper, when G₁ and G₃ represent a 2,2,2-trichloroethoxycarbonyl or 2-(2-trichloromethylpropoxy)carbonyl radical, or by treatment in acidic medium such as for example hydrochloric acid in solution in an aliphatic alcohol containing 1 to 3 carbon atoms (methanol, ethanol, propanol or isopropanol) or aqueous hydrofluoric acid at a temperature of between 0 and 40°C when G₃ represents silylated radical or an acetal residue, followed by the replacement of the protecting group G₁ by treatment using zinc, optionally combined with copper, in the presence of acetic acid at a temperature of between 30 and 60°C or by means of an inorganic or organic acid such as hydrochloric acid or acetic acid in solution in an aliphatic alcohol containing 1 to 3 carbon atoms or an aliphatic ester such as ethyl acetate, isopropyl acetate or n-butyl acetate in the presence of zinc optionally combined with copper, or, when G₁ represents an alkoxyacetyl radical, by treatment in alkaline medium by means of ammonia in aqueous-alcoholic medium at a temperature close to 20°C or by treatment using a zinc halide in methanol at a temperature close to 20°C.

30. Process according to Claim 25, **characterized in that** when G₃ represents a radical -CH₂-Ph, the replacement of the group by a hydrogen atom is carried out by hydrogenolysis, after replacing the protecting group G₁ under the conditions of Claim 29.

31. Process for the preparation of a product according to Claim 1, **characterized in that** an alkali metal halide or an alkali metal azide or a quaternary ammonium salt or an alkali metal phosphate is reacted with a product of general formula: 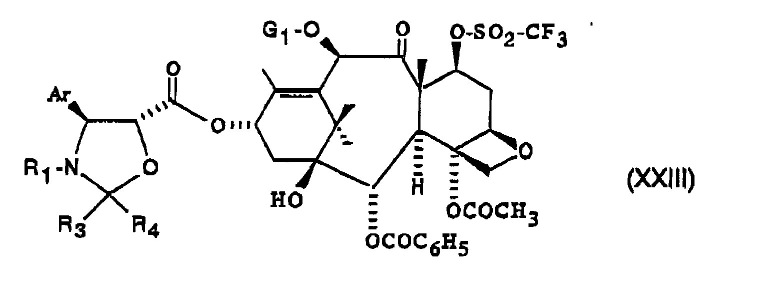 in which Ar and R₁ are defined as in one of Claims 1, 2 or 3 and G₁, R₃ and R₄ are as defined in either of Claims 6 and 14, in order to obtain a product of general formula: 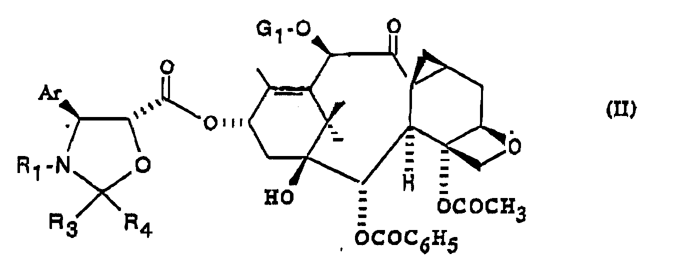 which is converted to the product according to one of Claims 1, 2 or 3, the process being carried out under the conditions of Claim 6.

32. Taxoid of general formula: 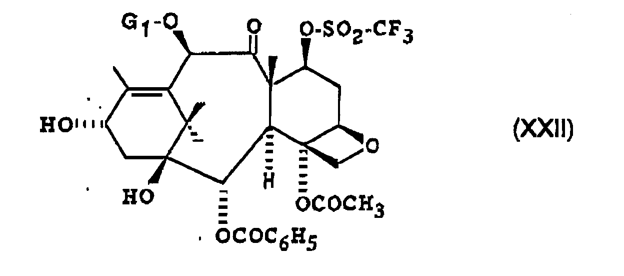 in which G₁ represents a hydrogen atom or an acetyl or alkoxyacetyl radical or a hydroxy-protecting group.

33. Process for the preparation of the derivative according to Claim 32 of general formula (XXII), **characterized in that** a trifluoroacetic acid derivative chosen from the anhydride or phenyltrifluoromethanesulphonimide is reacted with baccatin III or 10-deacetylbaccatin III.

34. Process according to Claim 33, **characterized, in that** the reaction is carried out in the presence of an organic base.

35. Process according to Claim 34, **characterized in that** the organic base is chosen from triethylamine and pyridine.

36. Taxoid of general formula (XXIII) : 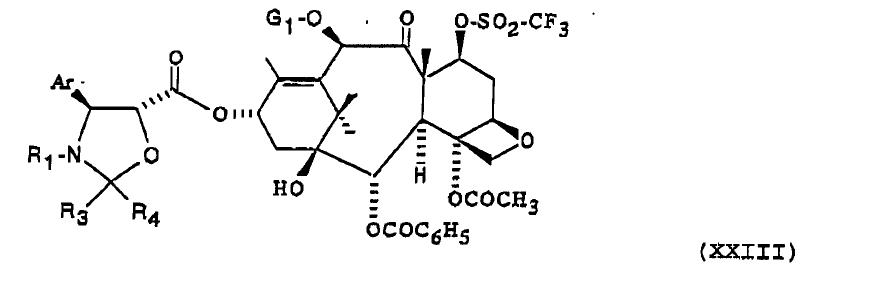 in which G₁ represents a hydrogen atom or an acetyl or alkoxyacetyl radical or a hydroxy-protecting group, Ar and R₁ are defined as in one of Claims 1 to 4 and R₃ and R₄ are defined as in either of Claims 6 and 14.

37. Process for the preparation of the product of general formula (XXIII) of Claim 36 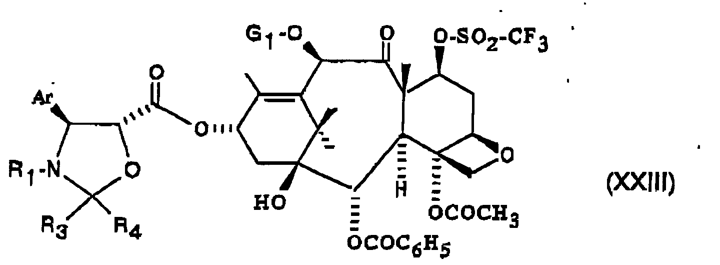 **characterized in that** a fluoromethanesulphonic acid derivative chosen from the anhydride or the N-phenyltrifluoromethanesulphinide is reacted with the product of general formula (XXIV): 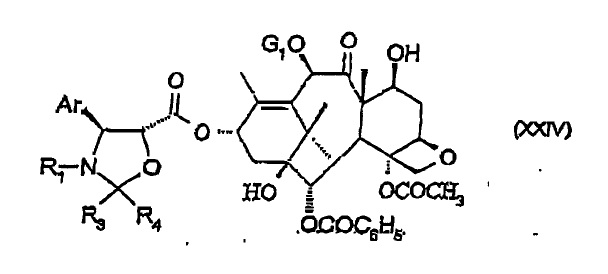 in which Ar, R₁, R₃ and R₄ are as defined above and G₁ represents a hydrogen atom or an acetyl radical.

38. Process according to Claim 37, **characterized in that** the reaction is carried out in an inert organic solvent in the presence of an organic base.

39. Process according to Claim 38, **characterized in that** the base is chosen from triethylamine or pyridine.

40. Process according to Claims 38 and 39, **characterized in that** the temperature is between -50 and +20°C.

41. Taxoid of general formula: 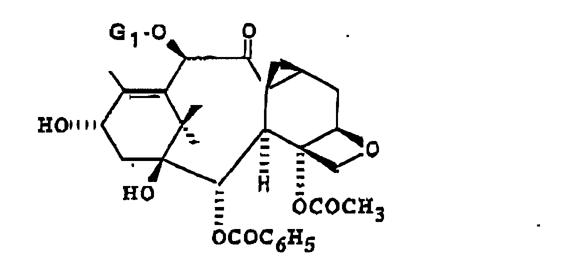 in which G₁ represents a hydrogen atom or an acetyl, alkoxyacetyl or alkyl radical or a hydroxy-protecting group.

42. Taxoids of formula (II) 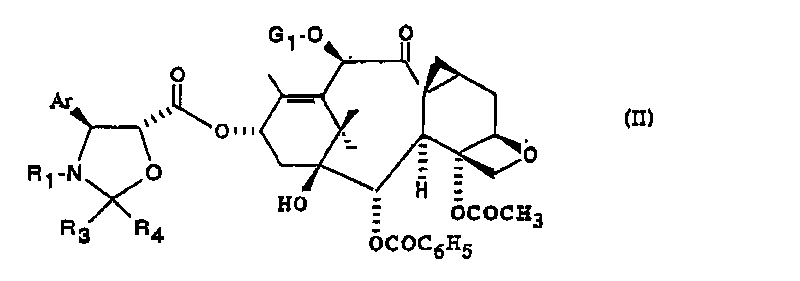 in which Ar, R₁, R₃, R₄ and G₁ are defined as in Claim 6.

43. Process for the preparation of the product of general formula (II) 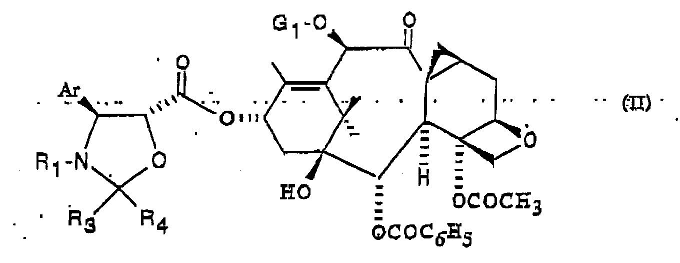 according to Claim 42, in which Ar, R₁, R₃ and R₄ are defined as in Claim 42 and G₁ represents a hydrogen atom or an acetyl radical, **characterized in that** the procedure is carried out starting with a product of general formula: 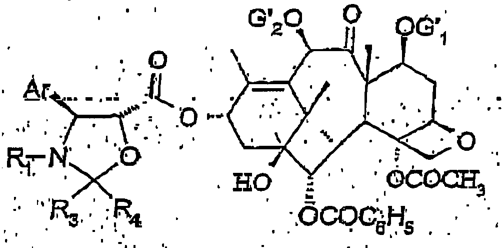 in which Ar, R₁, R₃ and R₄ are defined as in Claim 42, G'₁ represents a hydroxy-protecting group and G'₂ represents an acetyl, alkoxyacetyl or alkyl radical or a hydroxy-protecting group,
- by replacing the protecting groups G'₁ and optionally G'₂ by hydrogen atoms, in order to obtain the product of general formula: 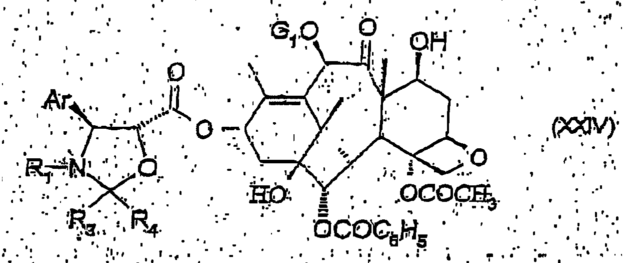 in which Ar, R₁, R₃ and R₄ are as defined above and G₁ represents a hydrogen atom or an acetyl radical,
- followed by the action of a trifluoromethanesulphonic acid derivative in order to obtain the product of general formula: 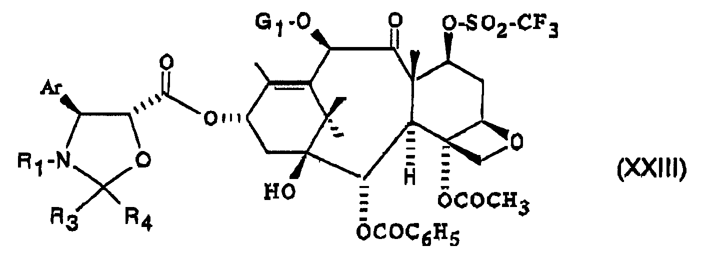
- and followed by the action of an alkali metal halide, or an alkali metal azide; or a quaternary ammonium salt or an alkali metal phosphate in order to obtain the product of general formula (II)

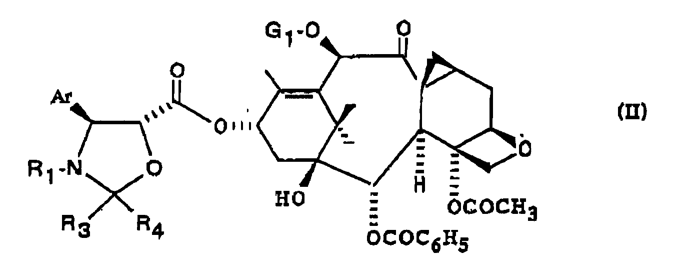

44. Process according to Claim 43, **characterized in that** the replacement of the protecting groups G'₁ and optionally G'₂ is carried out with zinc and acetic acid when G'₁ and G'₂ are both protecting groups.

45. Process according to Claim 43, **characterized in that** the replacement of G'₂ is carried out with hydrochloric acid in ethanolic solution when G'₂ is a silylated group.

46. Pharmaceutical composition, **characterized in that** it contains at least one product according to one of Claims 1 to 5, in combination with one or more pharmaceutically acceptable products, whether inert or physiologically active.

47. Use of the taxoids according to one of Claims 1 to 5 for the preparation of a medicament for the treatment of abnormal cell proliferation of malignant or nonmalignant cells of various tissues and/or organs, comprising muscle, bone or connective tissues, the skin, brain, lungs, sex organs, the lymphatic or renal systems, mammary or blood cells, liver, the digestive tract, pancreas and thyroid or adrenal glands and, for the treatment of the following pathological conditions: psoriasis, solid tumours, cancers of the ovary, breast, brain, prostate, colon, stomach, kidney or testicles, Kaposi's sarcoma, cholangioma, neuroblastoma, Wilms' tumour, Hodgkin's disease, melanomas, multiple myelomas, chronic lymphatic leukaemias and acute or chronic granulocytic lymphomas.
